# EUROPEAN PATENT APPLICATION

(11) **EP 4 813 976 A2**
(43) Date of publication of application: **30.09.2026**
(21) Application number: 26167345.3
(22) Date of filing: 24.03.2026
(51) Int. Cl.: C07D 327/10, C07D 497/04, H01M 6/00

(54) **ELECTROLYTE SOLUTION FOR ENERGY STORAGE DEVICE**

(30) Priority: 28.03.2025 US 202519094397
(71) Applicant: Tesla, Inc., Austin, TX 78725 (US)
(72) Inventor: MEISNER, Quinton James, Austin, 78725 (US); PETIBON, Remi, Austin, 78725 (US); AZAM, Saad Mohammad, Austin, 78725 (US); HY, Sunny, Austin, 78725 (US); DAHN, Jeffery Raymond, Austin, 78725 (US); YE, Ziwei, Austin, 78725 (US); METZGER, Michael, Austin, 78725 (US)
(74) Representative: Cousens, Nico

(57) **Abstract**

Various aspects disclosed relate to an electrolyte solution for an energy storage device.

## Description

### BACKGROUND

Energy storage devices, such as lithium-ion and sodium-ion batteries, can play a role in various applications including electric vehicles and portable electronics. The performance of these devices can be heavily influenced by the composition of their electrolyte solutions. Conventional electrolyte solutions can face challenges in achieving fast charging capabilities while maintaining long-term stability and safety.

### SUMMARY

The present disclosure provides electrolyte solutions for energy storage devices as well as methods of making and using the electrolyte solutions and devices incorporating the electrolyte solutions.

### BRIEF DESCRIPTION OF THE DRAWINGS

The drawings illustrate generally, by way of example, but not by way of limitation, various aspects of the present disclosure.
FIG. 1 shows a side cross-sectional schematic view of an example of an energy storage device.
FIG. 2 is a high-level diagram of an example vehicle.
FIG. 3 is a series of plots showing first cycle efficiency (FCE), gas generated during the formation cycle, first discharge capacity and full cell charge transfer resistance (Ret) of lithium ion batteries including various electrolyte solutions.
FIG. 4 is a series of plots showing voltage hysteresis (ΔV) (V), normalized capacity, and discharge capacity (mAh) of lithium ion batteries including various electrolyte solutions.
FIG. 5 is a series of plots showing discharge energy loss and ΔV growth of lithium ion batteries including various electrolyte solutions.

### DETAILED DESCRIPTION

Reference will now be made in detail to certain aspects of the disclosed subject matter, examples of which are illustrated in part in the accompanying drawings. While the disclosed subject matter will be described in conjunction with the enumerated claims, it will be understood that the exemplified subject matter is not intended to limit the claims to the disclosed subject matter.

Throughout this document, values expressed in a range format should be interpreted in a flexible manner to include not only the numerical values explicitly recited as the limits of the range, but also to include all the individual numerical values or sub-ranges encompassed within that range as if each numerical value and sub-range is explicitly recited. For example, a range of "about 0.1% to about 5%" or "about 0.1% to 5%" should be interpreted to include not just about 0.1% to about 5%, but also the individual values (e.g., 1%, 2%, 3%, and 4%) and the sub-ranges (e.g., 0.1% to 0.5%, 1.1% to 2.2%, 3.3% to 4.4%) within the indicated range. The statement "about X to Y" has the same meaning as "about X to about Y," unless indicated otherwise. Likewise, the statement "about X, Y, or about Z" has the same meaning as "about X, about Y, or about Z," unless indicated otherwise.

In this document, the terms "a," "an," or "the" are used to include one or more than one unless the context clearly dictates otherwise. The term "or" is used to refer to a nonexclusive "or" unless otherwise indicated. The statement "at least one of A and B" or "at least one of A or B" has the same meaning as "A, B, or A and B." In addition, it is to be understood that the phraseology or terminology employed herein, and not otherwise defined, is for the purpose of description only and not of limitation. Any use of section headings is intended to aid reading of the document and is not to be interpreted as limiting; information that is relevant to a section heading may occur within or outside of that particular section.

In the methods described herein, the acts can be carried out in any order without departing from the principles of the disclosure, except when a temporal or operational sequence is explicitly recited. Furthermore, specified acts can be carried out concurrently unless explicit claim language recites that they be carried out separately. For example, a claimed act of doing X and a claimed act of doing Y can be conducted simultaneously within a single operation, and the resulting process will fall within the literal scope of the claimed process.

The term "about" as used herein can allow for a degree of variability in a value or range, for example, within 10%, within 5%, or within 1% of a stated value or of a stated limit of a range, and includes the exact stated value or range. The term "substantially" as used herein refers to a majority of, or mostly, as in at least about 50%, 60%, 70%, 80%, 90%, 95%, 96%, 97%, 98%, 99%, 99.5%, 99.9%, 99.99%, or at least about 99.999% or more, or 100%. The term "substantially free of" as used herein can mean having none or having a trivial amount of, such that the amount of material present does not affect the material properties of the composition including the material, such that about 0 wt% to about 5 wt% of the composition is the material, or about 0 wt% to about 1 wt%, or about 5 wt% or less, or less than or equal to about 4.5 wt%, 4, 3.5, 3, 2.5, 2, 1.5, 1, 0.9, 0.8, 0.7, 0.6, 0.5, 0.4, 0.3, 0.2, 0.1, 0.01, or about 0.001 wt% or less, or about 0 wt%.

The term "organic group" as used herein refers to any carbon-containing functional group. Examples can include an oxygen-containing group such as an alkoxy group, aryloxy group, aralkyloxy group, oxo(carbonyl) group; a carboxyl group including a carboxylic acid, carboxylate, and a carboxylate ester; a sulfur-containing group such as an alkyl and aryl sulfide group; and other heteroatom-containing groups. Non-limiting examples of organic groups include OR, OOR, OC(O)N(R)₂, CN, CF₃, OCF₃, R, C(O), methylenedioxy, ethylenedioxy, N(R)₂, SR, SOR, SO₂R, SO₂N(R)₂, SO₃R, C(O)R, C(O)C(O)R, C(O)CH₂C(O)R, C(S)R, C(O)OR, OC(O)R, C(O)N(R)₂, OC(O)N(R)₂, C(S)N(R)₂, (CH₂)₀₋₂N(R)C(O)R, (CH₂)₀₋₂N(R)N(R)₂, N(R)N(R)C(O)R, N(R)N(R)C(O)OR, N(R)N(R)CON(R)₂, N(R)SO₂R, N(R)SO₂N(R)₂, N(R)C(O)OR, N(R)C(O)R, N(R)C(S)R, N(R)C(O)N(R)₂, N(R)C(S)N(R)₂, N(COR)COR, N(OR)R, C(=NH)N(R)₂, C(O)N(OR)R, C(=NOR)R, and substituted or unsubstituted (C₁-C₁₀₀)hydrocarbyl, wherein R can be hydrogen (in examples that include other carbon atoms) or a carbon-based moiety, and wherein the carbon-based moiety can be substituted or unsubstituted.

The term "substituted" as used herein in conjunction with a molecule or an organic group as defined herein refers to the state in which one or more hydrogen atoms contained therein are replaced by one or more non-hydrogen atoms. The term "functional group" or "substituent" as used herein refers to a group that can be or is substituted onto a molecule or onto an organic group. Examples of substituents or functional groups include, but are not limited to, a halogen (e.g., F, Cl, Br, and I); an oxygen atom in groups such as hydroxy groups, alkoxy groups, aryloxy groups, aralkyloxy groups, oxo(carbonyl) groups, carboxyl groups including carboxylic acids, carboxylates, and carboxylate esters; a sulfur atom in groups such as thiol groups, alkyl and aryl sulfide groups, sulfoxide groups, sulfone groups, sulfonyl groups, and sulfonamide groups; a nitrogen atom in groups such as amines, hydroxyamines, nitriles, nitro groups, N-oxides, hydrazides, azides, and enamines; and other heteroatoms in various other groups. Non-limiting examples of substituents that can be bonded to a substituted carbon (or other) atom include F, Cl, Br, I, OR, OC(O)N(R)₂, CN, NO, NO₂, ONO₂, azido, CF₃, OCF₃, R, O (oxo), S (thiono), C(O), S(O), methylenedioxy, ethylenedioxy, N(R)₂, SR, SOR, SO₂R, SO₂N(R)₂, SO₃R, C(O)R, C(O)C(O)R, C(O)CH₂C(O)R, C(S)R, C(O)OR, OC(O)R, C(O)N(R)₂, OC(O)N(R)₂, C(S)N(R)₂, (CH₂)₀₋₂N(R)C(O)R, (CH₂)₀₋₂N(R)N(R)₂, N(R)N(R)C(O)R, N(R)N(R)C(O)OR, N(R)N(R)CON(R)₂, N(R)SO₂R, N(R)SO₂N(R)₂, N(R)C(O)OR, N(R)C(O)R, N(R)C(S)R, N(R)C(O)N(R)₂, N(R)C(S)N(R)₂, N(COR)COR, N(OR)R, C(=NH)N(R)₂, C(O)N(OR)R, and C(=NOR)R, wherein R can be hydrogen or a carbon-based moiety; for example, R can be hydrogen, (C₁-C₁₀₀)hydrocarbyl, alkyl, acyl, cycloalkyl, aryl, aralkyl, heterocyclyl, heteroaryl, or heteroarylalkyl; or wherein two R groups bonded to a nitrogen atom or to adjacent nitrogen atoms can together with the nitrogen atom or atoms form a heterocyclyl.

The term "alkyl" as used herein refers to straight chain and branched alkyl groups and cycloalkyl groups having from 1 to 40 carbon atoms, 1 to about 20 carbon atoms, 1 to 12 carbons or, in some aspects, from 1 to 8 carbon atoms. Examples of straight chain alkyl groups include those with from 1 to 8 carbon atoms such as methyl, ethyl, n-propyl, n-butyl, n-pentyl, n-hexyl, n-heptyl, and n-octyl groups. Examples of branched alkyl groups include, but are not limited to, isopropyl, iso-butyl, sec-butyl, t-butyl, neopentyl, isopentyl, and 2,2-dimethylpropyl groups. As used herein, the term "alkyl" encompasses n-alkyl, isoalkyl, and anteisoalkyl groups as well as other branched chain forms of alkyl. Representative substituted alkyl groups can be substituted one or more times with any of the groups listed herein, for example, amino, hydroxy, cyano, carboxy, nitro, thio, alkoxy, and halogen groups.

The term "alkenyl" as used herein refers to straight and branched chain and cyclic alkyl groups as defined herein, except that at least one double bond exists between two carbon atoms. Thus, alkenyl groups have from 2 to 40 carbon atoms, or 2 to about 20 carbon atoms, or 2 to 12 carbon atoms or, in some aspects, from 2 to 8 carbon atoms. Examples include, but are not limited to vinyl, - CH=CH(CH₃), -CH=C(CH₃)₂, -C(CH₃)=CH₂, -C(CH₃)=CH(CH₃), - C(CH₂CH₃)=CH₂, cyclohexenyl, cyclopentenyl, cyclohexadienyl, butadienyl, pentadienyl, and hexadienyl among others.

The term "alkynyl" as used herein refers to straight and branched chain alkyl groups, except that at least one triple bond exists between two carbon atoms. Thus, alkynyl groups have from 2 to 40 carbon atoms, 2 to about 20 carbon atoms, or from 2 to 12 carbons or, in some aspects, from 2 to 8 carbon atoms. Examples include, but are not limited to -C≡CH, -C≡C(CH₃), - C≡C(CH₂CH₃), -CH₂C≡CH, -CH₂C≡C(CH₃), and -CH₂C≡C(CH₂CH₃) among others.

The term "acyl" as used herein refers to a group containing a carbonyl moiety wherein the group is bonded via the carbonyl carbon atom. The carbonyl carbon atom is bonded to a hydrogen forming a "formyl" group or is bonded to another carbon atom, which can be part of an alkyl, aryl, aralkyl cycloalkyl, cycloalkylalkyl, heterocyclyl, heterocyclylalkyl, heteroaryl, heteroarylalkyl group or the like. An acyl group can include 0 to about 12, 0 to about 20, or 0 to about 40 additional carbon atoms bonded to the carbonyl group. An acyl group can include double or triple bonds within the meaning herein. An acryloyl group is an example of an acyl group. An acyl group can also include heteroatoms within the meaning herein. A nicotinoyl group (pyridyl-3-carbonyl) is an example of an acyl group within the meaning herein. Other examples include acetyl, benzoyl, phenylacetyl, pyridylacetyl, cinnamoyl, and acryloyl groups and the like. When the group containing the carbon atom that is bonded to the carbonyl carbon atom contains a halogen, the group is termed a "haloacyl" group. An example is a trifluoroacetyl group.

The term "cycloalkyl" as used herein refers to cyclic alkyl groups such as, but not limited to, cyclopropyl, cyclobutyl, cyclopentyl, cyclohexyl, cycloheptyl, and cyclooctyl groups. In some aspects, the cycloalkyl group can have 3 to about 8-12 ring members, whereas in other aspects the number of ring carbon atoms range from 3 to 4, 5, 6, or 7. Cycloalkyl groups further include polycyclic cycloalkyl groups such as, but not limited to, norbornyl, adamantyl, bornyl, camphenyl, isocamphenyl, and carenyl groups, and fused rings such as, but not limited to, decalinyl, and the like. Cycloalkyl groups also include rings that are substituted with straight or branched chain alkyl groups as defined herein. Representative substituted cycloalkyl groups can be mono-substituted or substituted more than once, such as, but not limited to, 2,2-, 2,3-, 2,4- 2,5- or 2,6-disubstituted cyclohexyl groups or mono-, di- or tri-substituted norbornyl or cycloheptyl groups, which can be substituted with, for example, amino, hydroxy, cyano, carboxy, nitro, thio, alkoxy, and halogen groups. The term "cycloalkenyl" alone or in combination denotes a cyclic alkenyl group.

The term "aryl" as used herein refers to cyclic aromatic hydrocarbon groups that do not contain heteroatoms in the ring. Thus aryl groups include, but are not limited to, phenyl, azulenyl, heptalenyl, biphenyl, indacenyl, fluorenyl, phenanthrenyl, triphenylenyl, pyrenyl, naphthacenyl, chrysenyl, biphenylenyl, anthracenyl, and naphthyl groups. In some aspects, aryl groups contain about 6 to about 14 carbons in the ring portions of the groups. Aryl groups can be unsubstituted or substituted, as defined herein. Representative substituted aryl groups can be mono-substituted or substituted more than once, such as, but not limited to, a phenyl group substituted at any one or more of 2-, 3-, 4-, 5-, or 6-positions of the phenyl ring, or a naphthyl group substituted at any one or more of 2-to 8-positions thereof.

The term "aralkyl" as used herein refers to alkyl groups as defined herein in which a hydrogen or carbon bond of an alkyl group is replaced with a bond to an aryl group as defined herein. Representative aralkyl groups include benzyl and phenylethyl groups and fused (cycloalkylaryl)alkyl groups such as 4-ethyl-indanyl. Aralkenyl groups are alkenyl groups as defined herein in which a hydrogen or carbon bond of an alkyl group is replaced with a bond to an aryl group as defined herein.

The term "heterocyclyl" as used herein refers to aromatic and nonaromatic ring compounds containing three or more ring members, of which one or more is a heteroatom such as, but not limited to, N, O, and S.

The term "heteroaryl" as used herein refers to aromatic ring compounds containing 5 or more ring members, of which one or more is a heteroatom such as, but not limited to, N, O, and S; for instance, heteroaryl rings can have 5 to about 8-12 ring members. A heteroaryl group is a variety of a heterocyclyl group that possesses an aromatic electronic structure.

The term "heterocyclylalkyl" as used herein refers to alkyl groups as defined herein in which a hydrogen or carbon bond of an alkyl group as defined herein is replaced with a bond to a heterocyclyl group as defined herein. Representative heterocyclyl alkyl groups include, but are not limited to, furan-2-yl methyl, furan-3-yl methyl, pyridine-3-yl methyl, tetrahydrofuran-2-yl ethyl, and indol-2-yl propyl.

The term "heteroarylalkyl" as used herein refers to alkyl groups as defined herein in which a hydrogen or carbon bond of an alkyl group is replaced with a bond to a heteroaryl group as defined herein.

The term "alkoxy" as used herein refers to an oxygen atom connected to an alkyl group, including a cycloalkyl group, as are defined herein. Examples of linear alkoxy groups include but are not limited to methoxy, ethoxy, propoxy, butoxy, pentyloxy, hexyloxy, and the like. Examples of branched alkoxy include but are not limited to isopropoxy, sec-butoxy, tert-butoxy, isopentyloxy, isohexyloxy, and the like. Examples of cyclic alkoxy include but are not limited to cyclopropyloxy, cyclobutyloxy, cyclopentyloxy, cyclohexyloxy, and the like. An alkoxy group can include about 1 to about 12, about 1 to about 20, or about 1 to about 40 carbon atoms bonded to the oxygen atom, and can further include double or triple bonds, and can also include heteroatoms. For example, an allyloxy group or a methoxyethoxy group is also an alkoxy group within the meaning herein, as is a methylenedioxy group in a context where two adjacent atoms of a structure are substituted therewith.

The term "amine" as used herein refers to primary, secondary, and tertiary amines having, e.g., the formula N(group)₃ wherein each group can independently be H or non-H, such as alkyl, aryl, and the like. Amines include but are not limited to R-NH₂, for example, alkylamines, arylamines, alkylarylamines; R₂NH wherein each R is independently selected, such as dialkylamines, diarylamines, aralkylamines, heterocyclylamines and the like; and R₃N wherein each R is independently selected, such as trialkylamines, dialkylarylamines, alkyldiarylamines, triarylamines, and the like. The term "amine" also includes ammonium ions as used herein.

The term "amino group" as used herein refers to a substituent of the form -NH₂, -NHR, -NR₂, -NR₃⁺, wherein each R is independently selected, and protonated forms of each, except for -NR₃⁺, which cannot be protonated. Accordingly, any compound substituted with an amino group can be viewed as an amine. An "amino group" within the meaning herein can be a primary, secondary, tertiary, or quaternary amino group. An "alkylamino" group includes a monoalkylamino, dialkylamino, and trialkylamino group.

The terms "halo," "halogen," or "halide" group, as used herein, by themselves or as part of another substituent, mean, unless otherwise stated, a fluorine, chlorine, bromine, or iodine atom.

The term "haloalkyl" group, as used herein, includes mono-halo alkyl groups, poly-halo alkyl groups wherein all halo atoms can be the same or different, and per-halo alkyl groups, wherein all hydrogen atoms are replaced by halogen atoms, such as fluoro. Examples of haloalkyl include trifluoromethyl, 1,1-dichloroethyl, 1,2-dichloroethyl, 1,3-dibromo-3,3-difluoropropyl, perfluorobutyl, and the like.

The term "monovalent" as used herein refers to a substituent connecting via a single bond to a substituted molecule. When a substituent is monovalent, such as, for example, F or Cl, it is bonded to the atom it is substituting by a single bond.

The term "hydrocarbon" or "hydrocarbyl" as used herein refers to a molecule or functional group that includes carbon and hydrogen atoms. The term can also refer to a molecule or functional group that normally includes both carbon and hydrogen atoms but wherein all the hydrogen atoms are substituted with other functional groups. The term "hydrocarbyl" refers to a functional group derived from a straight chain, branched, or cyclic hydrocarbon, and can be alkyl, alkenyl, alkynyl, aryl, cycloalkyl, acyl, or any combination thereof. Hydrocarbyl groups can be shown as (Cₐ-C_{b})hydrocarbyl, wherein a and b are integers and mean having any of a to b number of carbon atoms. For example, (C₁-C₄)hydrocarbyl means the hydrocarbyl group can be methyl (C₁), ethyl (C₂), propyl (C₃), or butyl (C₄), and (C₀-C_{b})hydrocarbyl means in certain aspects there is no hydrocarbyl group. A hydrocarbylene group is a diradical hydrocarbon, e.g., a hydrocarbon that is bonded at two locations.

Various aspects according to the instant disclosure are directed towards an electrolyte solution which can be used in an energy storage device such as a battery (e.g., a lithium-ion battery). The electrolyte solution includes a solvent component, an additive component, and an alkali salt.

In the electrolyte solution, the solvent component can range from about 70 wt% to about 95 wt% of the total electrolyte mass, about 75 wt% to about 85 wt%, less than, equal to, or greater than about 75 wt%, 76, 77, 78, 79, 80, 81, 82, 83, 84, 85, 86, 87, 88, 89, 90, 91, 92, 93, 94, or about 95 wt% of the total electrolyte mass. The solvent component can include a linear ester, a cyclic organic carbonate, a linear organic carbonate, or a mixture of at least two thereof. Examples of linear esters can include methyl acetate, ethyl acetate, and methyl propionate. Examples of cyclic organic carbonates can include ethylene carbonate and propylene carbonate, fluoroethylene carbonate, di-fluoroethylene carbonate, and vinylene carbonate. Examples of linear organic carbonates can include dimethyl carbonate, methyl ethyl carbonate, and diethyl carbonate.

If present, the linear ester can range from 0.01 wt% to about 100 wt% of the solvent component, about 5 wt% to about 70 wt%, about 5 wt% to about 50 wt%, about 8 wt% to about 30 wt%, less than, equal to, or greater than about 0 wt%, 5, 10, 15, 20, 25, 30, 35, 40, 45, 50, 55, 60, 65, 70, 75, 80, 85, 90, 95, or about 100 wt%. If present, the cyclic organic carbonate can range from about 0.01 wt% to about 100 wt% of the solvent component, about 5 wt% to about 40 wt%, about 5 wt% to about 25 wt%, less than, equal to, or greater than about 0 wt%, 5, 10, 15, 20, 25, 30, 35, 40, 45, 50, 55, 60, 65, 70, 75, 80, 85, 90, 95, or about 100 wt%. If present, the linear organic carbonate can range from about 0.01 wt% to about 100 wt% of the solvent component, about 2 wt% to about 95 wt%, about 5 wt% to about 60 wt%, less than, equal to, or greater than about 0 wt%, 5, 10, 15, 20, 25, 30, 35, 40, 45, 50, 55, 60, 65, 70, 75, 80, 85, 90, 95, or about 100 wt%.

In some examples (e.g., in a lithium ion battery), the alkali salt can include a lithium salt. Examples of suitable alkali salts can include lithium hexafluorophosphate (LiPF₆), lithium bis(fluorosulfonyl)imide (LiFSI), lithium bis-oxalatoborate (LiBOB), lithium difluorooxalatoborate (LiDFOB), lithium bis(trifluoromethanesulfonyl)imide (LiTFSI), or lithium bis(trifluoromethanesulfonyl)imide (LiTFSI), or a mixture of at least two thereof. In some examples (e.g., in a sodium-ion battery), the alkali salt can include a sodium salt. Examples of suitable sodium salts can include sodium bis(fluorosulfonyl)imide (NaFSi), sodium bis-trifluoromethansulfonate (NaTFSi),sodium hexafluorophosphate (NaPF6) or a mixture of at least two thereof. The total alkali salt concentration in the electrolyte solution can range from about 0.5m to about 1.8m, about 0.8m to about 1.4m, less than, equal to, or greater than about 0.5m, 0.6, 0.7, 0.8, 0.9, 1, 1.1, 1.2, 1.3, 1.4, 1.5, 1.6, 1.7, or about 1.8.

The additive component can range from about 0.01 wt% to about 9 wt% of the electrolyte solution, about 0.5 wt% to about 2 wt%, less than, equal to, or greater than about 0.01 wt%, 0.05, 0.10, 0.15, 0.20, 0.25, 0.30, 0.35, 0.40, 0.45, 0.50, 0.55, 0.60, 0.65, 0.70, 0.75, 0.80, 0.85, 0.90, 0.95, 1, 1.05, 1.10, 1.15, 1.20, 1.25, 1.30, 1.35, 1.40, 1.45, 1.50, 1.55, 1.60, 1.65, 1.70, 1.75, 1.80, 1.85, 1.90, 1.95, 2, 2.05, 2.10, 2.15, 2.20, 2.25, 2.30, 2.35, 2.40, 2.45, 2.50, 2.55, 2.60, 2.65, 2.70, 2.75, 2.80, 2.85, 2.90, 2.95, 3 3.05, 3.10, 3.15, 3.20, 3.25, 3.30, 3.35, 3.40, 3.45, 3.50, 3.55, 3.60, 3.65, 3.70, 3.75, 3.80, 3.85, 3.90, 3.95, 4, 4.05, 4.10, 4.15, 4.20, 4.25, 4.30, 4.35, 4.40, 4.45, 4.50, 4.55, 4.60, 4.65, 4.70, 4.75, 4.80, 4.85, 4.90, 4.95, or about 5 wt%.

The additive component can include a compound having a structure according to formula (I): 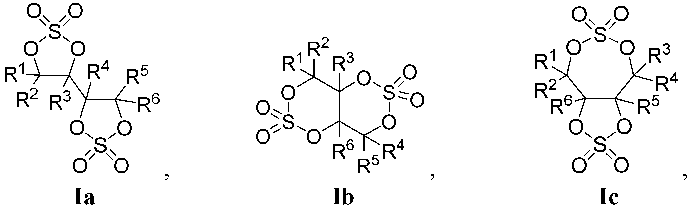 or 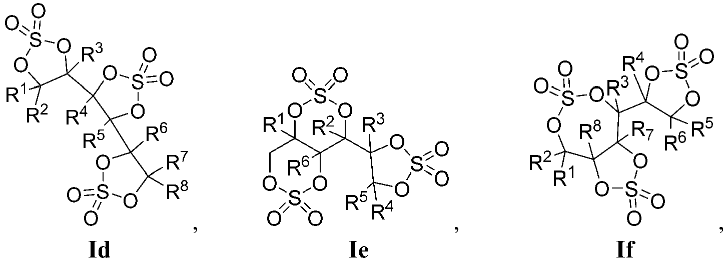 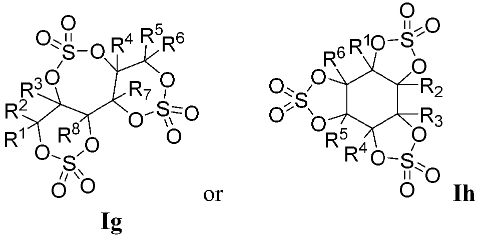 At each occurrence, R¹-R⁸ are independently H, a halogen, substituted or unsubstituted C₁-C₂₀ hydrocarbyl, or substituted or unsubstituted C₁-C₂₀ haloalkyl. In some examples, at least one of or each of R¹-R⁸ is H. In some examples at least one of, or each of, R¹-R⁸ is a halogen. In some examples, the halogen is F. In some examples, at least one of, or each of, R¹-R⁸ is substituted or unsubstituted C₁-C₂₀ alkyl. In some examples, at least one of, or each of, R¹-R⁸ is substituted or unsubstituted C₁-C₂₀ aryl. In some examples, at least one of, or each of R¹-R⁸ are substituted or unsubstituted C₁-C₂₀ haloalkyl. In some examples, at least one of, or each or, R¹-R⁸ are substituted or unsubstituted C₁-C₂₀ fluoroalkyl.

Examples of suitable structures of formula (I) include the following: 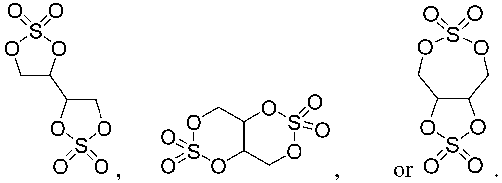 The additive component can further include any enantiomer, diastereomer, *meso* compound, or constitutional isomer of the structure of formula (I). Although the instant disclosure contemplates and includes many possible enantiomers, diastereomers, *meso* compounds, or constitutional isomers of the structure of formula (I), the following are presented as non-limiting examples:

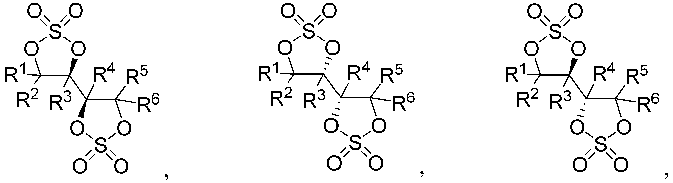

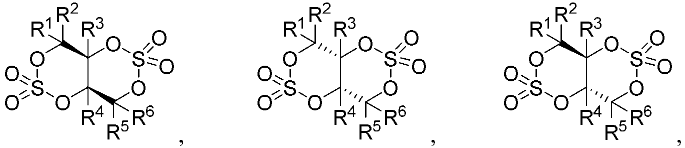

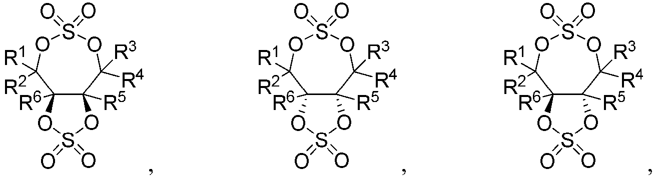

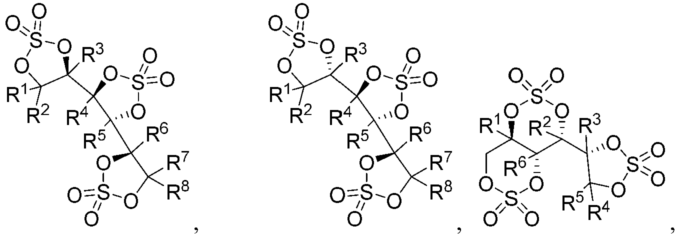

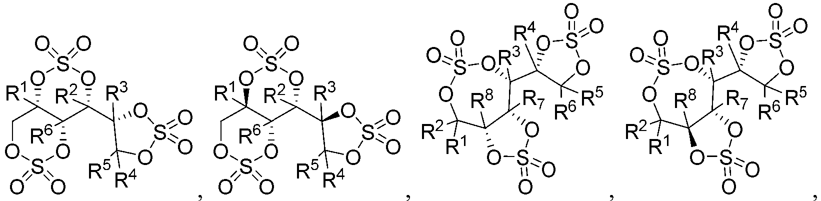

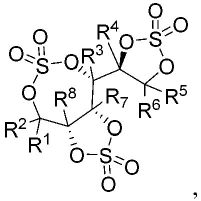

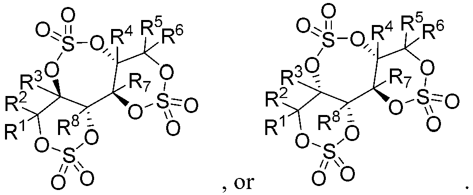

In some examples, the structure of formula (I) can include the following structures:

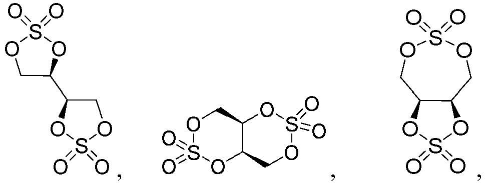

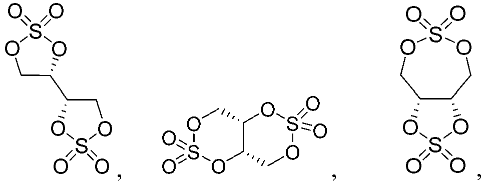

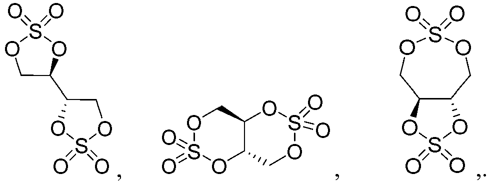

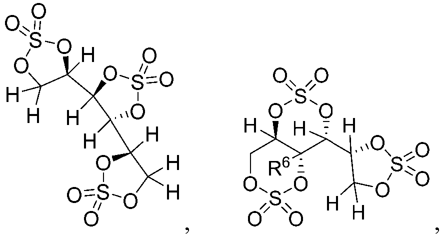

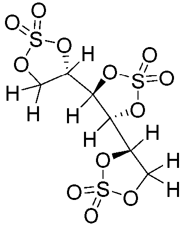

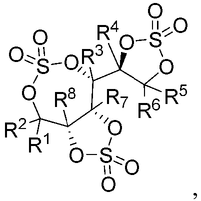

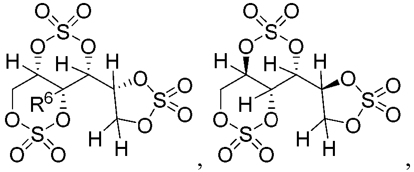

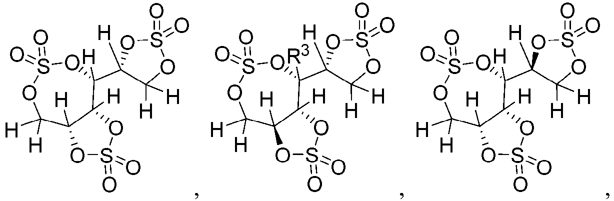

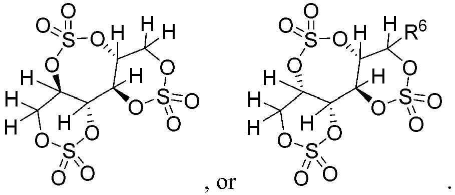

The additive component can include any one of the aforementioned structures. Additionally, the additive component can include a mixture of at least two of the aforementioned structures, at least three of the aforementioned structures, or other plural number. In examples, where the additive component is a mixture of the aforementioned structures, each structure can individually account for about 0.05 wt% to about 99 wt% of the additive component, about 5 wt% to about 80 wt%, about 10 wt% to about 60 wt%, less than, equal to, or greater than about 0.05 wt%, 0.5, 1, 5, 10, 15, 20, 25, 30, 35, 40, 45, 50, 55, 60, 65, 70, 75, 80, 85, 90, 95, or about 99 wt%.

Additional additives that can be included as a mixture with any of the aforementioned additives include Vinylene carbonate (VC), Vinyl ethylene carbonate (VEC), Fluoroethylene carbonate (FEC), 1,3,2-Dioxathiolane 2,2-Dioxide (DTD), 1-Propene 1,3-Sultone (PRS, or PES), 1,3 Propanesultone (PS), Lithium difluorophosphate (LFO), Lithium bisoxalatodifluorophosphate (LiBODFP), Lithium tetrafluoro(oxalato)phosphate (LiTFOP), Tris(trimethylsilyl) Phosphate (TMSP), Tris(trimethylsilyl) phosphite (TTSPi), Sodium difluorophosphate (NaFO), Sodium bisoxalatodifluorophosphate (NaBOB), Sodium tetrafluoro(oxalato)phosphate (NaTFOP), or a mixture of at least two thereof.

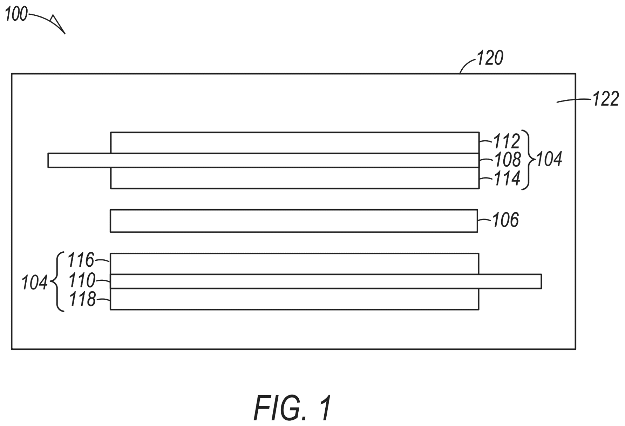

FIG. 1 shows a side cross-sectional schematic view of an example of an energy storage device 100 to which the aforementioned electrolyte solution can be incorporated. The energy storage device 100 may be a lithium-ion battery or a sodium-ion battery. It should be realized that other energy storage devices are within the scope of the disclosure, and can include batteries, capacitor-battery hybrids, and/or fuel cells. The energy storage device 100 can have a first electrode 102, a second electrode 104, and a separator 106 positioned between the first electrode 102 and second electrode 104. For example, the first electrode 102 and the second electrode 104 may be placed adjacent to respective opposing surfaces of the separator 106. The first electrode 102 may comprise a cathode and the second electrode 104 may comprise an anode, or vice versa. The energy storage device 100 includes the aforementioned electrolyte solution 122 to facilitate ionic communication between the electrodes 102, 104 of the energy storage device 100. For example, the electrolyte solution 122 may be in contact with the first electrode 102, the second electrode 104, and the separator 106.

The electrolyte solution 122, the first electrode 102, the second electrode 104, and the separator 106 may be received within an energy storage device housing 120. For example, the energy storage device housing 120 may be sealed subsequent to insertion of the first electrode 102, the second electrode 104 and the separator 106, and impregnation of the energy storage device 100 with the electrolyte solution 122, such that the first electrode 102, the second electrode 104, the separator 106, and the electrolyte solution may be physically sealed from an environment external to the housing. The electrolyte solution 122 optionally includes one or more additives as provided herein.

The separator 106 can be configured to electrically insulate two electrodes adjacent to opposing sides of the separator 106, such as the first electrode 102 and the second electrode 104, while permitting ionic communication between the two adjacent electrodes. The separator 106 can comprise a variety of porous electrically insulating materials. In some aspects, the separator 106 can include a polymeric material, a glass fiber, a ceramic-coated polymer, or a combination of at least two thereof. For example, the separator 106 can comprise polyethylene (PE), polypropylene (PP), polyethylene-polypropylene multi-layer (PP/PE/PP), polyvinylidene fluoride (PVDF), anodized alumina (AAO), polyethylene oxide (PEO), polyvinyl alcohol (PVA), β"-alumina, Na₃Zr₂Si₂PO₁₂ (NASICON), and zeolite A.

As shown in FIG. 1, the first electrode 102 and the second electrode 104 include a first current collector 108 and a second current collector 110, respectively. The first current collector 108 and the second current collector 110 may facilitate electrical coupling between the corresponding electrode and an external circuit (not shown). The first current collector 108 and/or the second current collector 110 can include one or more electrically conductive materials, and/or have various shapes and/or sizes configured to facilitate transfer of electrical charges between the corresponding electrode and a terminal for coupling the energy storage device 100 with an external terminal, including an external electrical circuit. For example, a current collector can include a metallic material, such as a material comprising aluminum, nickel, copper, silver, alloys thereof, and/or the like. For example, the first current collector 108 and/or the second current collector 110 can comprise an aluminum foil having a rectangular or substantially rectangular shape and can be dimensioned to provide desired transfer of electrical charges between the corresponding electrode and an external electrical circuit (e.g., via a current collector plate and/or another energy storage device component configured to provide electrical communication between the electrodes and the external electrical circuit).

The first electrode 102 may have a first electrode film 112 (e.g., an upper electrode film) on a first surface of the first current collector 108 (e.g., on a top surface of the first current collector 108) and a second electrode film 114 (e.g., a lower electrode film) on a second opposing surface of the first current collector 108 (e.g., on a bottom surface of the first current collector 108). Similarly, the second electrode 104 may have a first electrode film 116 (e.g., an upper electrode film) on a first surface of the second current collector 110 (e.g., on a top surface of the second current collector 110), and a second electrode film 118 on a second opposing surface of the second current collector 110 (e.g., on a bottom surface of the second current collector 110). For example, the first surface of the second current collector 110 may face the second surface of the first current collector 108, such that the separator 106 is adjacent to the second electrode film 114 of the first electrode 102 and the first electrode film 116 of the second electrode 104.

The electrode films 112, 114, 116 and/or 118 can have a variety of suitable shapes, sizes, and/or thicknesses. For example, the electrode films can have a thickness of about 30 microns (µm) to about 250 microns, including about 100 microns to about 250 microns. It will be understood that aspects described herein can be implemented with one or more electrodes, and with electrode(s) that have one or more electrode films, and should not be limited to the aspect shown in FIG. 1.

In some aspects, an electrode film of an anode and/or a cathode of a lithium-ion battery or sodium-ion battery comprises a mixture comprising binder material and carbon. Examples of suitable cathode materials can include LiNi_{0.42}Mn_{0.42}Co_{0.16}O₂ (NMC442), LiNi_{0.65}Mn_{0.15}Co_{0.20}O₂ (Ni65), LiNi_{0.91}Mn_{0.045}Co_{0.045}O₂ (Ni91), LiNi_{0.63}Mn_{0.31}Co_{0.06}O₂ (NM64), and LiNiₓCo_{y}Al_{1-x-y}O₂ where x + y + z = 1 (NCA), and LiNiₓMn_{y}Co_{1-x-y}O₂ (NMC) where x + y + z = 1, LiFePO₄ (LFP), LiFeₓMn₁₋ₓPO₄ (LMFP), LiNiₓMn₂₋ₓO₄ (LNMO), LiMn₂O₄ (LMO), and NaNiₓFe_{y}Mn_{z}O₂, where x + y + z = 1 (NFM). Examples of suitable anode materials can include graphite; hard carbon; a Si material comprising Si, SiO, LiSiOₓ, SiC or any combination of graphite and Si material; Sn or any combination of hard carbon and Sn, Li₄Ti₅O₁₂ (LTO); Na₂Ti₆O₁₃ (NTO); and NaTi₂(PO₄)₃ (NTP).

In some aspects, the electrode film of an anode and/or a cathode can include one or more additives, including conductive additives. In some aspects, the binder material can include one or more fibrillizable binder components. For example, a process for forming an electrode film can include fibrillizing the fibrillizable binder component such that the electrode film comprises fibrillized binder. The binder component may be fibrillized to provide a plurality of fibrils, the fibrils providing desired mechanical support for one or more other components of the film. For example, a matrix, lattice and/or web of fibrils can be formed to provide desired mechanical structure for the electrode film. For example, a cathode and/or an anode of a lithium-ion or sodium-ion capacitor can include one or more electrode films comprising one or more fibrillized binder components. In some aspects, a binder component can include one or more of a variety of suitable fibrillizable polymeric materials, such as polytetrafluoroethylene (PTFE), ultra-high molecular weight polyethylene (UHMWPE), and/or other suitable fibrillizable materials, used alone or in combination.

In some aspects, the electrode film of a lithium-ion or sodium-ion capacitor cathode can comprise an electrode film mixture comprising one or more carbon based electroactive components, including for example a porous carbon material, such as activated carbon. In some aspects, the electrode film of a lithium-ion or sodium-ion capacitor anode comprises an electrode film mixture comprising carbon configured to reversibly intercalate lithium ions or sodium ions, respectively. In some aspects, the lithium intercalating carbon is graphite. In some aspects, the electrode film of the cathode and/or anode can include an electrical conductivity promoting additive, for example, comprising carbon black.

In some aspects, an energy storage device, such as device 100, can be fabricated by a method comprising providing a cathode and providing an anode, such as electrodes 102, 104; placing a separator, such as separator 106, between the cathode and the anode, inserting the cathode, the anode, and the separator into a housing, such as housing 120, and adding the electrolyte solution 122, to the housing, and contacting the electrolyte with the cathode and the anode.

The energy storage device 100 including the aforementioned electrolyte solution can be incorporated in portable applications, vehicles, domestic buildings, commercial building, or grid energy storage. Examples of vehicles include a car, passenger vehicle, truck, bus, shuttle, motorcycle, scooter, utility vehicle, construction vehicle, mining vehicle, agricultural vehicle, aircraft, plane, helicopter, vertical takeoff and landing (eVTOL) vehicle, drone, unmanned aerial vehicle (UAV), airship, space vehicle, boat, ship, ferry, personal watercraft, submersible, marine vessel, port vehicle, train, tram, light rail vehicle, or subway car.

An operating temperature of the energy storage device can be in a range of from about -40 to about 90 °C, about -30 to about 75 °C, less than, equal to, or greater than about -40 °C, -35, -30, -25, -20, -15, -10, -5, 0, 5, 10, 15, 20, 25, 30, 35, 40, 45, 50, 55, 60, 65, 70, 75, 80, 85, or about 95 °C.

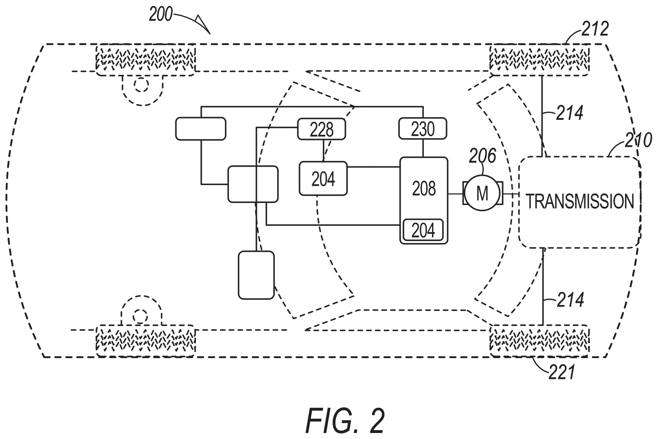

FIG. 2 is a high-level diagram of a vehicle 200 that can include energy storage device 100. In various aspects, the vehicle 200 is an electric vehicle and includes a vehicle propulsion energy storage device 204, including the aforementioned electrolyte solution 122, and at least one propulsion motor 206 for converting battery energy into mechanical motion, such as rotary motion. The present subject matter includes examples in which the energy storage device 204 is a subcomponent of an energy storage system ("ESS"). An ESS includes various components associated with transmitting energy to and from the energy storage device 204 in various examples, including safety components, cooling components, heating components, rectifiers, etc.

A state of charge circuit 228 is pictured to monitor the state of charge of the energy storage device 204 . The state of charge circuit 228 can count coulombs, watt-hours, or provide other measure of how much energy is in the energy storage device 204 . In some aspects, the state of charge is determined by measuring the battery voltage either open circuited or driving a known load. In additional aspects, the state of charge circuit could optionally provide additional battery information, such as temperature, rate of energy use, number of charge/discharge cycles, and other information relating to battery state. The state of charge circuit 228 can be integrated into an ESS.

Additionally illustrated is an energy converter 208. The energy converter 208 is part of a system which converts energy from the vehicle propulsion energy storage device 204 into energy useable by the at least one propulsion motor 206. In certain instances, the energy flow is from the at least one propulsion motor 206 to the energy storage device 204. As such, in some examples, the energy storage device 204 transmits energy to the energy converter 208, which converts the energy into energy usable by the at least one propulsion motor 206 to propel the electric vehicle. In additional examples, the at least one propulsion motor 206 generates energy that is transmitted to the energy converter 208. In these examples, the energy converter 208 converts the energy into energy which can be stored in the energy storage device 204 .

Some examples of the energy converter 208 include one or more field effect transistors. Some examples include metal oxide semiconductor field effect transistors. Some examples include one more insulated gate bipolar transistors. As such, in various examples, the energy converter 208 includes a switch bank which is configured to receive a direct current ("DC") power signal from the energy storage device 204 and to output a three-phase alternating current ("AC") signal to power the vehicle propulsion motor 206. In some examples, the energy converter 208 is configured to convert a three-phase signal from the vehicle propulsion motor 206 to DC power to be stored in the energy storage device 204 . Some examples of the energy converter 208 convert energy from the energy storage device 204 into energy usable by electrical loads other than the vehicle propulsion motor 206. Some of these examples switch energy from approximately 390 volts DC to 14 volts DC.

The propulsion motor 206 is, in some aspects, a three phase alternating current ("AC") propulsion motor, in various examples. Some examples include a plurality of such motors. The present subject matter can optionally include a transmission or gearbox 210 in certain examples. While some examples include a 1-speed transmission, other examples are contemplated. Manually clutched transmissions are contemplated, as are those with hydraulic, electric, or electrohydraulic clutch actuation. Some examples employ a dual-clutch system that, during shifting, phases from one clutch coupled to a first gear to another coupled to a second gear. Rotary motion is transmitted from the transmission 210 to wheels 212 and 221via one or more axles 214, in various examples.

A fuel burning engine 230 is pictured though not a required component (e.g., for a fully electric vehicle). The fuel burning engine 230 can burn any of a variety of fuels, such as fossil fuels, synthetics and biofuels. The fuel burning engine 230 can be controlled to provide a continuous level of power or can provide a variable level of power via control such as throttle control, as is known. Some aspects operate the fuel burning engine 230 at a level which provides for the highest fuel efficiency.

The fuel burning engine 230 is shown connected to the energy converter 208, such that the fuel burning engine 230 could turn a generator to which it is coupled and provide power to the energy converter 208, but other configurations are possible. For example, in some aspects, an integrated engine generator provides power directly to the energy storage device 204 .

The engine can run constantly, but various examples turn the engine on or off based on energy requirements of the vehicle 200. External power is provided to communicate energy with the energy storage device 204, with a reduced overall charging time as discussed above. In various aspects, external power 218 includes a charging station that is coupled to a municipal power grid. In certain examples, the charging station converts power from a 110V AC power source into power storable by the energy storage device 204 . In additional examples, the charging station converts power from a 120V AC power source into power storable by the energy storage device 204. Some aspects include converting energy from the energy storage device 204 into power usable by a municipal grid. The present subject matter is not limited to examples in which a converter for converting energy from an external source to energy usable by the vehicle 200 is located outside the vehicle 200, and other examples are contemplated.

### Examples

Various aspects of the present disclosure can be better understood by reference to the following Examples which are offered by way of illustration. The present disclosure is not limited to the Examples given herein.

### EXAMPLE 1

A series of electrolyte solutions were prepared and evaluated in a lithium-ion battery having a layered cathode of LiNi_{0.63}Mn_{0.31}Co_{0.06}O₂ (NM64) for their first cycle efficiency (FCE) (%), Formation gas (mL), first discharge capacity (Ah), full cell charge transfer resistance (Rct) (Ω Ah), voltage hysteresis (ΔV) (V), normalized capacity, discharge capacity (mAh), discharge energy loss (%), and ΔV growth. Table 1 shows the composition of the electrolyte solutions.

**Table 1**

| **Solution** | **Ethylene carbonate (wt%)** | **Dimethyl carbonate (wt%)** | **Methyl acetate (wt%)** | **Fluoroethylene carbonate (wt%)** | **Vinylene ethylene carbonate (VEC) (wt%)** | **Bis-DTD (wt%)** | **1-Propene 1,3-Sultone (PRS) (wt%)** | **Lithium hexafluorophosphate (wt%)** | **Lithium bis(fluorosulfonyl) imide (wt%)** |
|---|---|---|---|---|---|---|---|---|---|
| Baseline (PRS) | 11.986 | 51.941 | 15.982 | 2 | 1 | - | 0.75 | 6.99 | 9.35 |
| bis-DTD | 11.955 | 51.806 | 15.94 | 2 | 1 | 1 | - | 6.972 | 9.326 |
| bis-DTD | 11.831 | 51.267 | 15.774 | 2 | 1 | 2 | - | 6.899 | 9.229 |

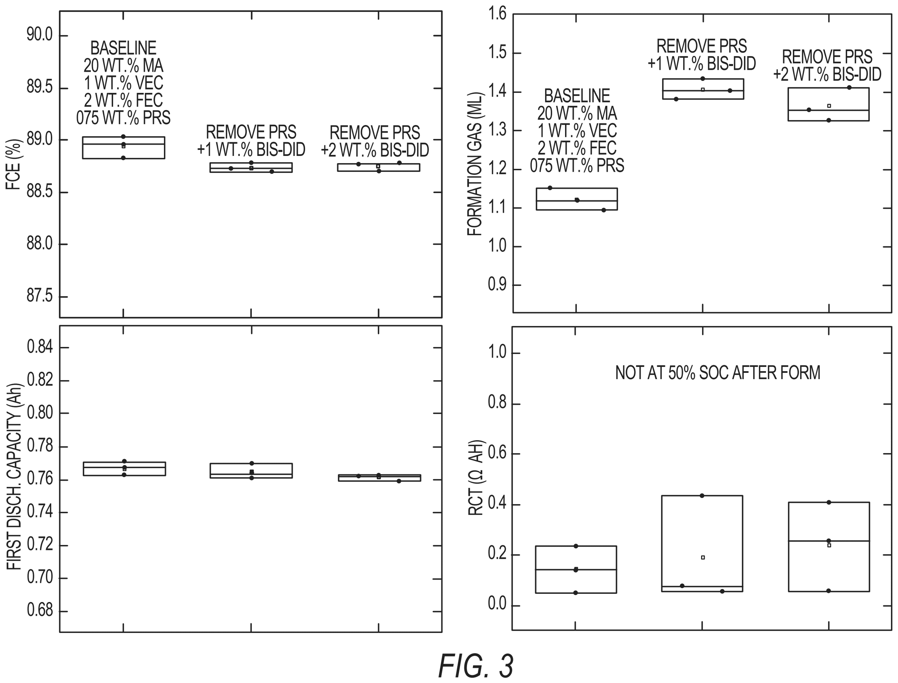

FIG. 3 is a series of plots showing a comparison of the two bis-DTD formulations versus the baseline formulation of Table 1. As shown, the bis-DTD formulations demonstrated slightly lower FCE (< 0.05%), a higher gassing of 0.3 mL (27%), and a comparable 1^{st} discharge.

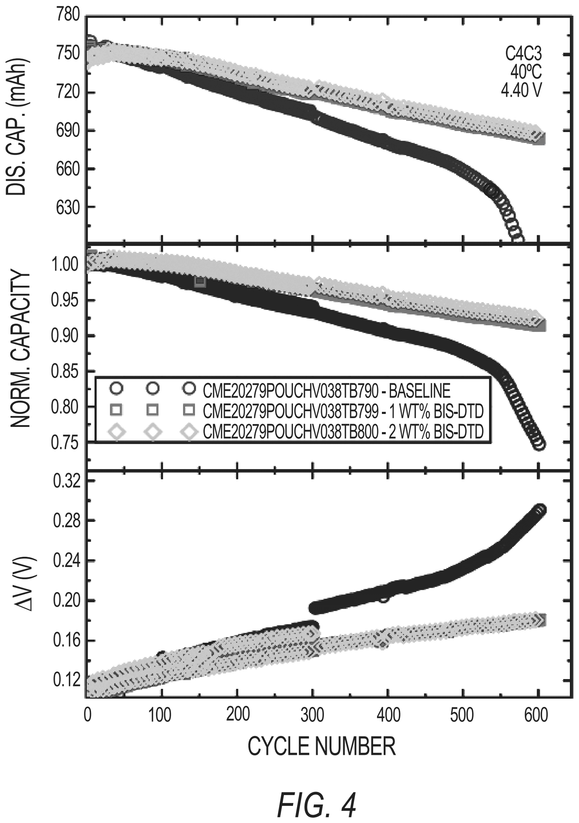

FIG. 4 is a series of plots showing a comparison of the two bis-DTD formulations versus the baseline formulation of Table 1. As shown, the bis-DTD shows improved capacity retention (> 92% @500 cyc, 40% slower loss rate) relative to the baseline formulation. Additionally, the bis-DTD shows reduced dV (< 0.17V @500 cyc).

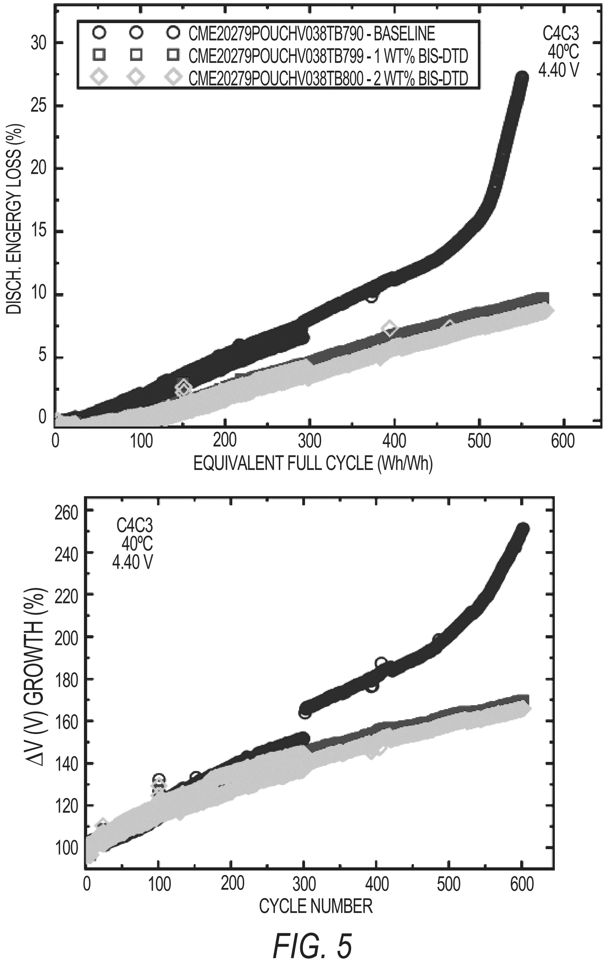

FIG. 5 is a series of plots showing a comparison of the two bis-DTD formulations versus the baseline formulation of Table 1. As shown, the two bis-DTD formulations show improved energy loss (< 8% @500cyc, 40% slower loss rate) and reduced dV growth (< 60 % @500 cyc).

### EXAMPLE 2

Example 2 provides a two-step synthetic scheme for production bis-DTD. Although a particular stereochemistry is depicted it is within the scope of the disclosure for other stereochemistry to be achieved.

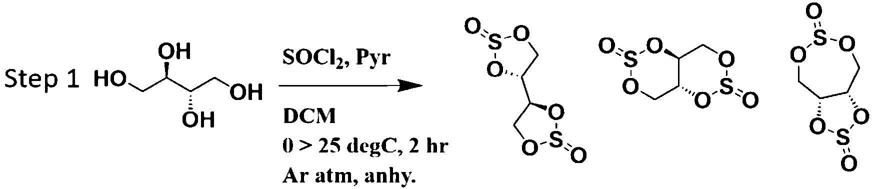

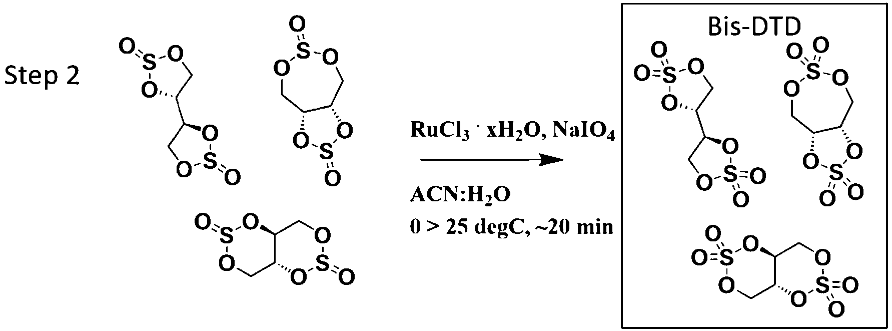

The terms and expressions that have been employed are used as terms of description and not of limitation, and there is no intention in the use of such terms and expressions of excluding any equivalents of the features shown and described or portions thereof, but it is recognized that various modifications are possible within the scope of the aspects of the present disclosure. Thus, it should be understood that although the present disclosure has been specifically disclosed by specific aspects and optional features, modification and variation of the concepts herein disclosed may be resorted to by those of ordinary skill in the art, and that such modifications and variations are considered to be within the scope of aspects of the present disclosure.

### Exemplary Aspects.

The following exemplary aspects are provided, the numbering of which is not to be construed as designating levels of importance:
Aspect 1 provides a compound having a structure according to formula (I): 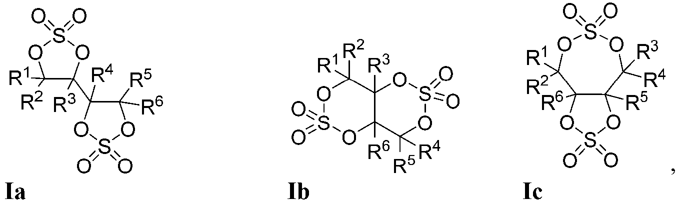 or 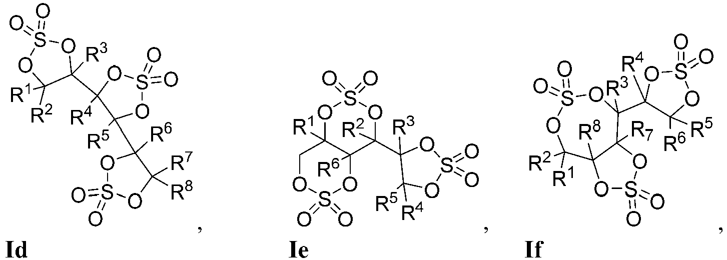 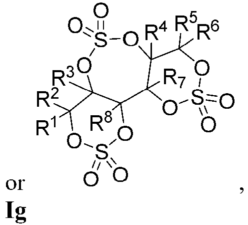 or 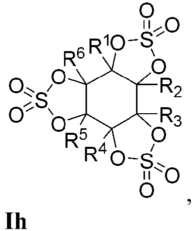
   wherein R¹-R⁸ are each independently H, a halogen, substituted or unsubstituted C₁-C₂₀ hydrocarbyl, or substituted or unsubstituted C₁-C₂₀ haloalkyl,
   or any enantiomer, diastereomer, *meso* compound, or constitutional isomer thereof.
Aspect 2 provides a compound of Aspect 1 having the structure: 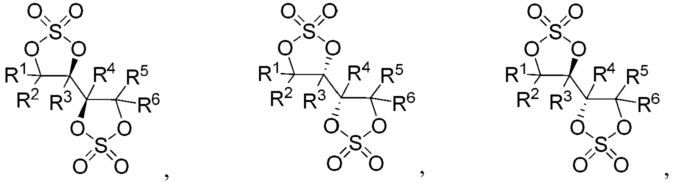 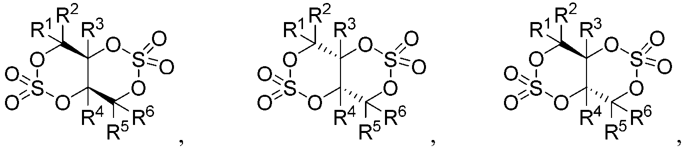 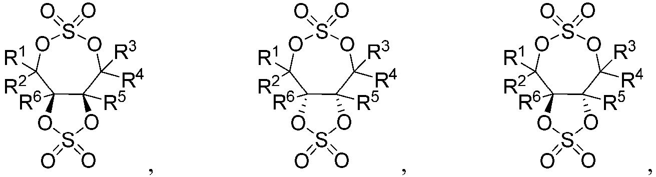 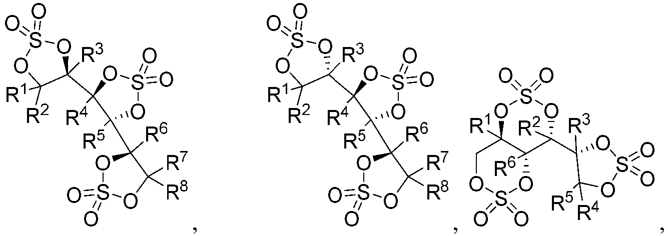 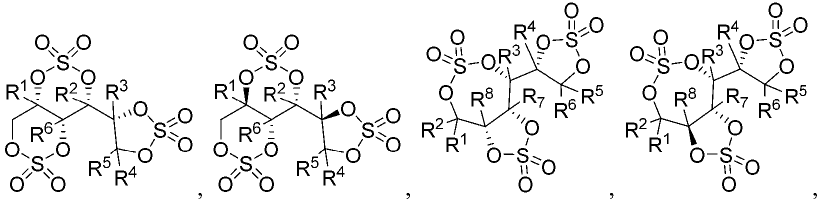 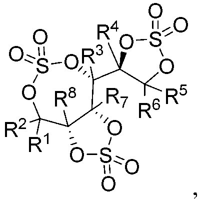 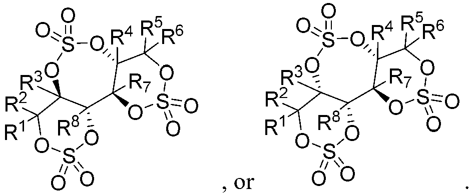
Aspect 3 provides a compound of Aspects 1 or 2, wherein at least one of R¹- R⁸ is H.
Aspect 4 provides a compound of Aspects 1 or 2, wherein at least one of R¹- R⁸ is a halogen.
Aspect 5 provides a compound of Aspect 4, wherein the halogen is F.
Aspect 6 provides a compound of Aspect 1 or 2, wherein at least one of R¹- R⁸ is substituted or unsubstituted C₁-C₂₀ alkyl.
Aspect 7 provides a compound of Aspect 1 or 2, wherein at least one of R¹- R⁸ is substituted or unsubstituted C₁-C₂₀ aryl.
Aspect 8 provides a compound of Aspect 1 or 2, wherein at least one of R¹- R⁸ are substituted or unsubstituted C₁-C₂₀ haloalkyl.
Aspect 9 provides a compound of Aspect 8, wherein at least one of R¹- R⁸ are substituted or unsubstituted C₁-C₂₀ fluoroalkyl.
Aspect 10 provides a compound of any of Aspects 1-3, wherein the compound has the structure: 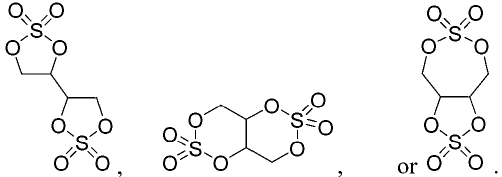
Aspect 11 provides a compound of Aspect 10, wherein the compound has the structure: 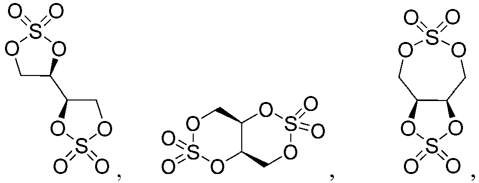 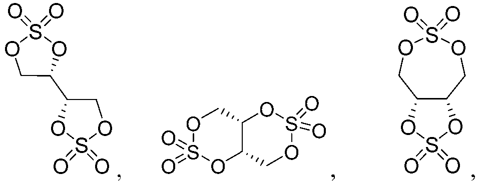 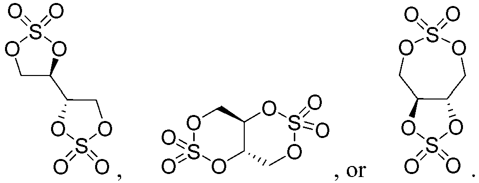
Aspect 12 provides a compound of Aspect 1 having the structure: 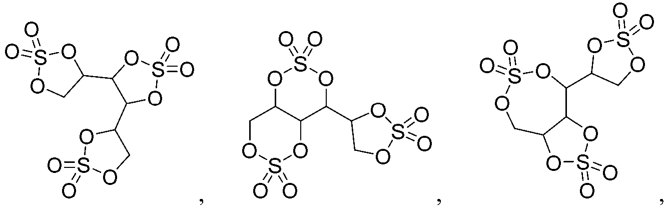 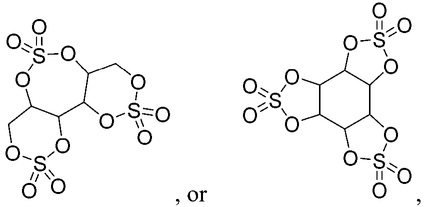 or any enantiomer, diastereomer, *meso* compound, or constitutional isomer thereof.
Aspect 13 provides a mixture comprising a compound or at least three compounds of any of Aspects 1-12.
Aspect 14 provides a mixture of Aspect 13 comprising compounds each having the structure: 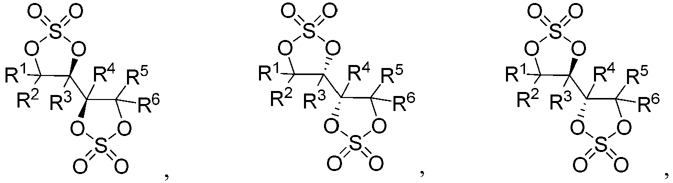 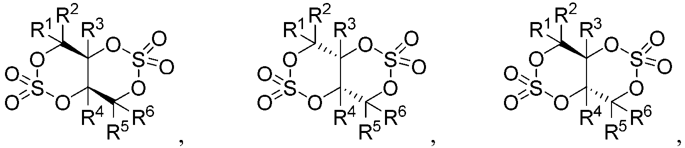 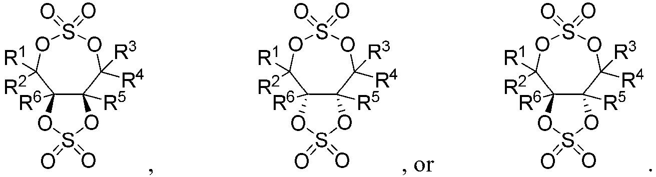
Aspect 15 provides a mixture of Aspect 14 comprising any three of the compounds 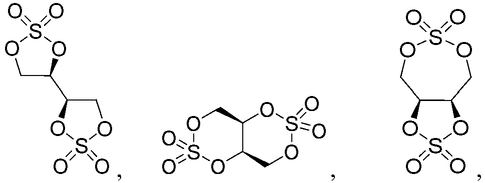 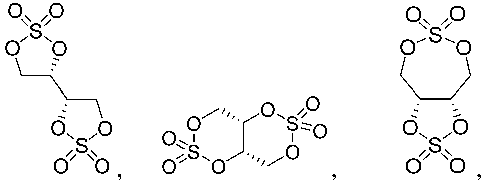 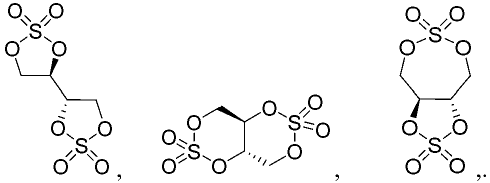 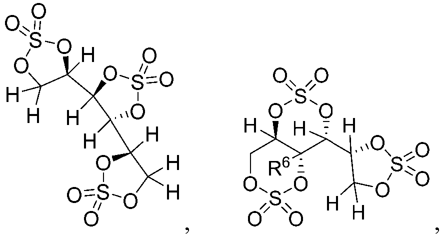 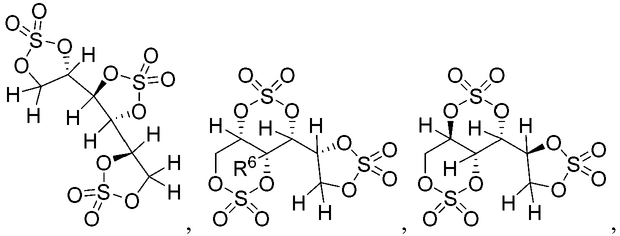 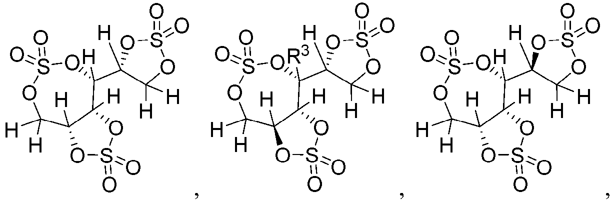 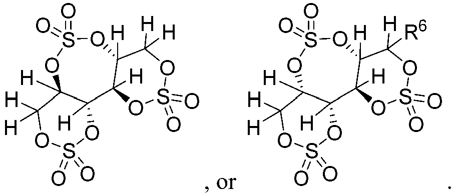
Aspect 16 provides an electrolyte solution comprising:
   a solvent comprising:
   at least one of:
   a linear ester;
   a cyclic organic carbonate;
   a linear organic carbonate; and
   an additive component, and
   an alkali salt.
Aspect 17 provides an electrolyte solution of Aspect 16, wherein at least one of
   the linear ester is selected from a group comprising methyl acetate, ethyl acetate, and methyl propionate,
   the cyclic organic carbonate is selected from a group comprising ethylene carbonate and propylene carbonate, fluoroethylene carbonate, di-fluoroethylene carbonate, and vinylene carbonate; and
   the linear organic carbonate is selected from a group comprising dimethyl carbonate, methyl ethyl carbonate, and diethyl carbonate.
1. Aspect 18 provides an electrolyte solution of Aspect 16 or 17, wherein the additive component comprises a compound having a structure according to formula (I): 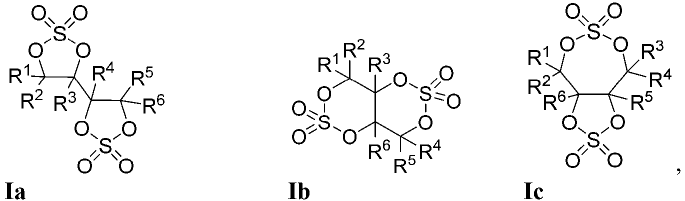 or 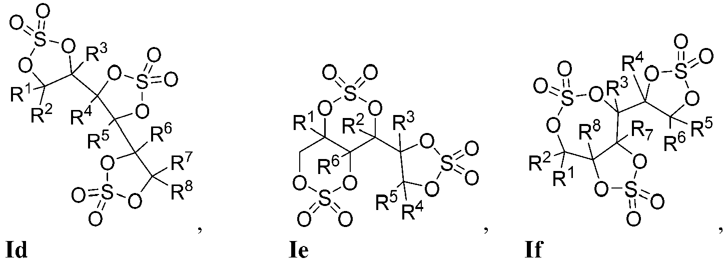 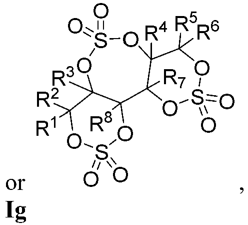 or 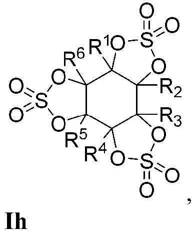
   wherein R¹- R⁸ are each independently H, a halogen, substituted or unsubstituted C₁-C₂₀ hydrocarbyl, or substituted or unsubstituted C₁-C₂₀ haloalkyl;
   or any enantiomer, diastereomer, *meso* compound, or constitutional isomer thereof.
Aspect 19 provides an electrolyte solution of Aspect 18, wherein the compound has the structure: 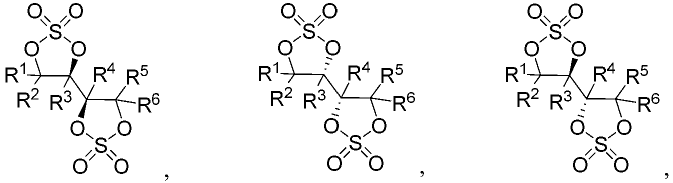 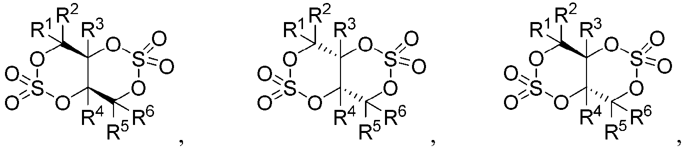 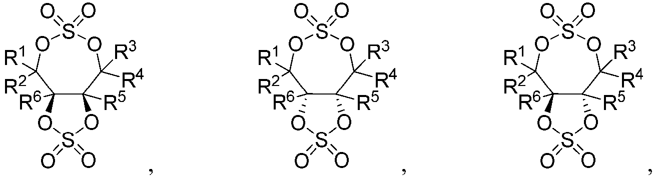 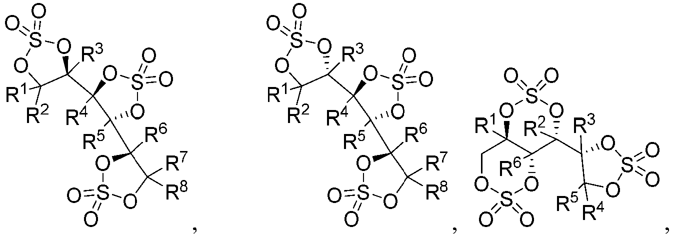 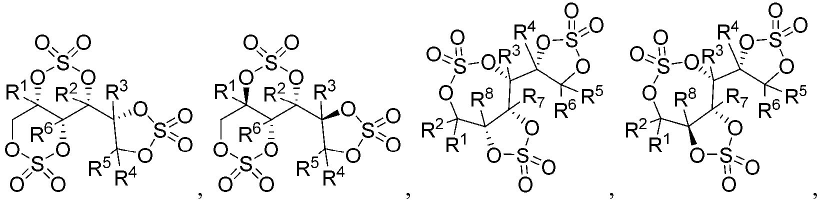 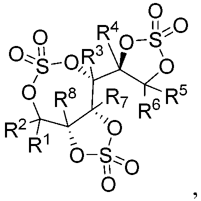 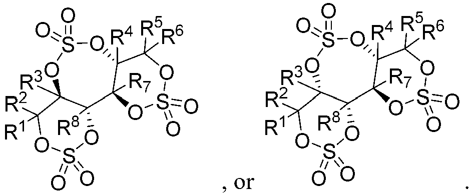
Aspect 20 provides an electrolyte solution of Aspect 18 or 19, wherein at least one of R¹- R⁸ is H.
Aspect 21 provides an electrolyte solution of Aspect 18 or 19, wherein at least one of R¹- R⁸ is a halogen.
Aspect 22 provides an electrolyte solution of Aspect 21, wherein the halogen is F.
Aspect 23 provides an electrolyte solution of Aspect 18 or 19, wherein at least one of R¹- R⁸ is substituted or unsubstituted C₁-C₂₀ alkyl.
Aspect 24 provides an electrolyte solution of Aspect 18 or 19, wherein at least one of R¹- R⁸ is substituted or unsubstituted C₁-C₂₀ aryl.
Aspect 25 provides an electrolyte solution of Aspect 18 or 19, wherein at least one of R¹- R⁸ are substituted or unsubstituted C₁-C₂₀ haloalkyl.
Aspect 26 provides an electrolyte solution of Aspect 25, wherein at least one of R¹- R⁸ are substituted or unsubstituted C₁-C₂₀ fluoroalkyl.
Aspect 27 provides an electrolyte solution of Aspect 20 comprising a compound having the structure: 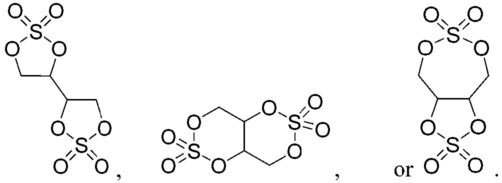
Aspect 28 provides an electrolyte solution of Aspect 27 comprising a compound having the structure: 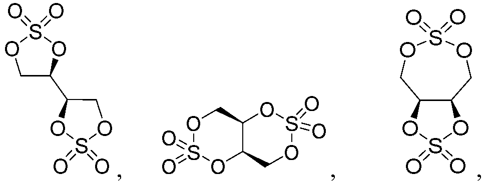 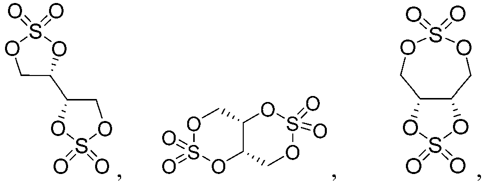 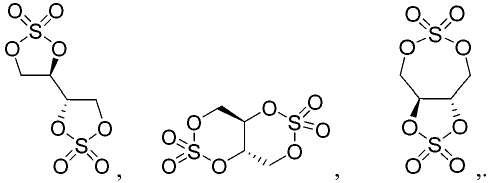 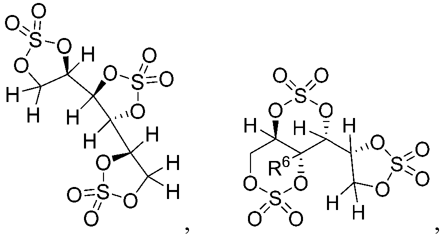 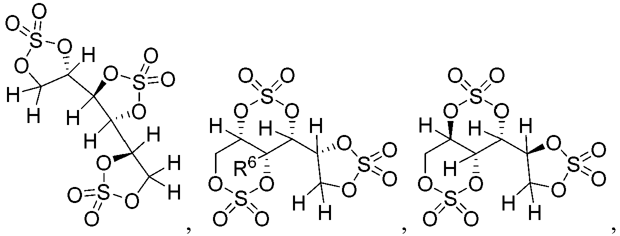 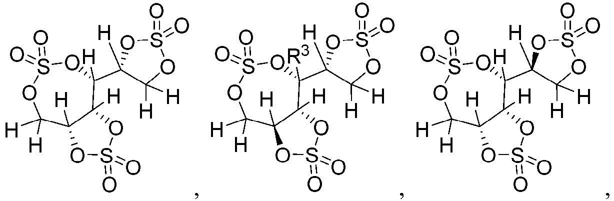 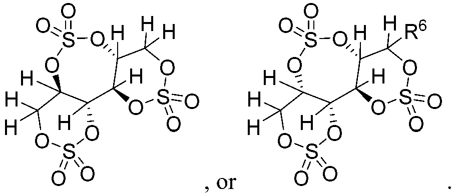
2. Aspect 29 provides an electrolyte solution of any of Aspects 16-28, wherein the additive component comprises a mixture of compounds each having a structure according to formula (I): 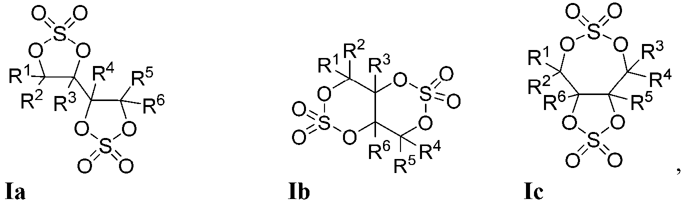 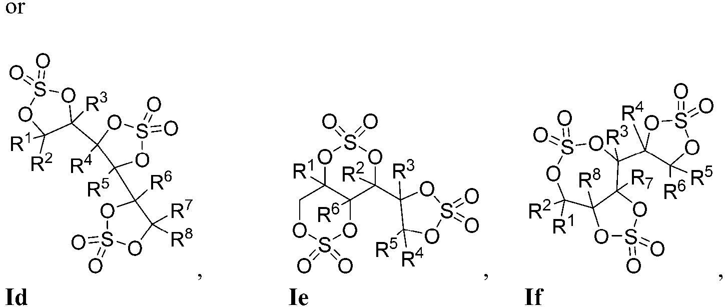 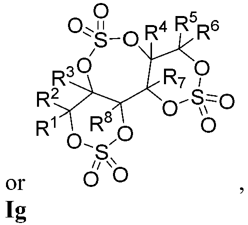 or 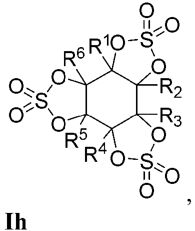 wherein R¹- R⁸ are each independently H, a halogen, substituted or unsubstituted C₁-C₂₀ hydrocarbyl, or substituted or unsubstituted C₁-C₂₀ haloalkyl or any enantiomer, diastereomer, *meso* compound, or constitutional isomer thereof.
Aspect 30 provides an electrolyte solution of Aspect 29, wherein the additive component comprises a mixture of compounds each having a structure: 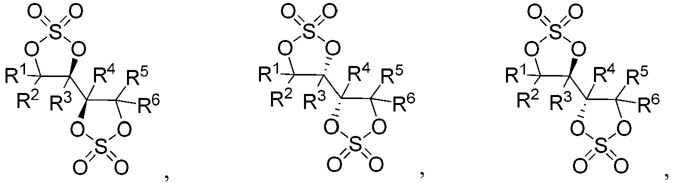 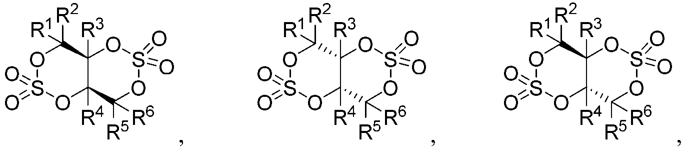 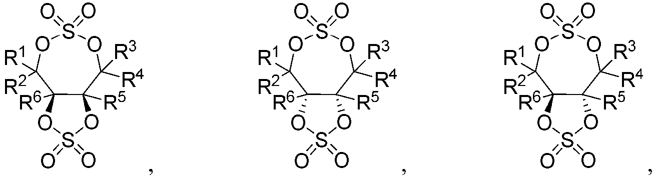 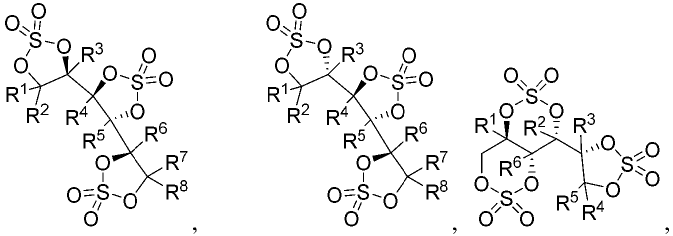 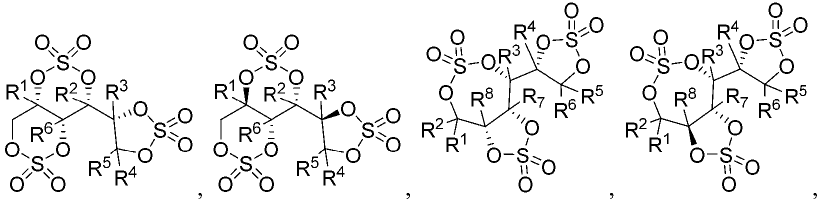 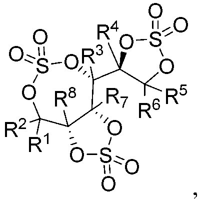 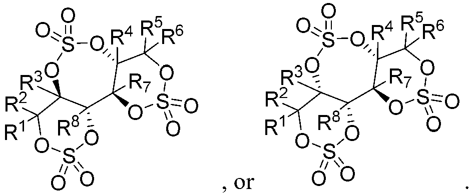
Aspect 31 provides an electrolyte solution of Aspect 30, wherein the additive component comprises a mixture of compounds each having the structure: 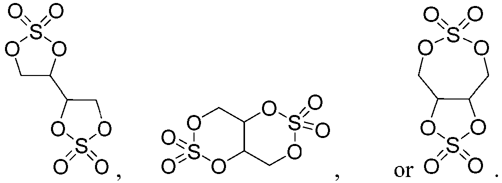
Aspect 32 provides an electrolyte solution of Aspect 31, wherein the additive component comprises a mixture of compounds each having the structure:
Aspect 33 provides an electrolyte solution of Aspect 32, wherein the additive component comprises a mixture of at least any three of the compounds: 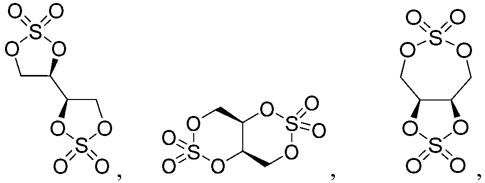 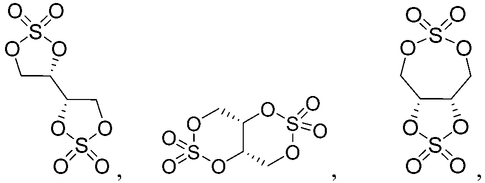 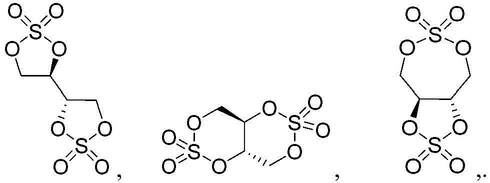 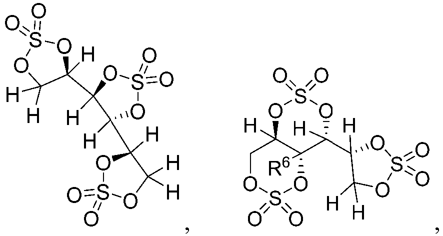 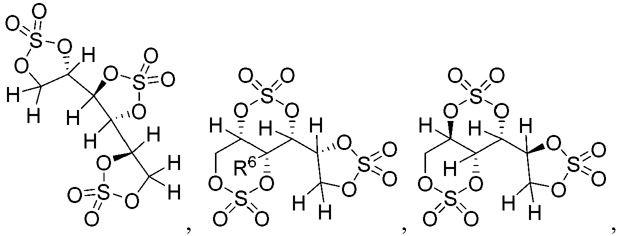 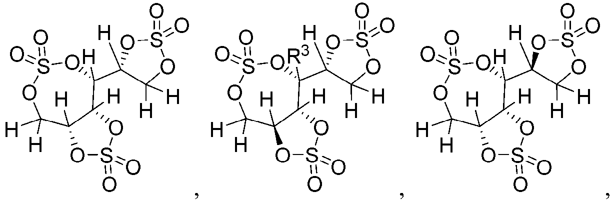 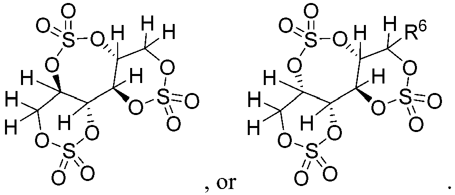
Aspect 34 provides an electrolyte solution of any of Aspects 16-33, wherein the alkali salt comprises lithium hexafluorophosphate (LiPF₆), lithium bis(fluorosulfonyl)imide (LiFSi), lithium bis-oxalatoborate (LiBOB), lithium difluorooxalatoborate (LiDFOB), lithium bis-trifluoromethansulfonate (LiTFSi), sodium hexafluorophosphate (NaPF₆), sodium bis(fluorosulfonyl)imide (NaFSi), sodium bis-trifluoromethansulfonate (NaTFSi), or a mixture thereof.
Aspect 35 provides an electrolyte solution of any of Aspects 16-34, wherein a linear ester concentration is in a range of from about 0 wt% to about 80 wt% of the solvent.
Aspect 36 provides an electrolyte solution of Aspect 35, wherein the linear ester concentration is in a range of from about 5 wt% to about 50 wt% of the solvent.
Aspect 37 provides an electrolyte solution of Aspect 36, wherein the linear ester concentration is in a range of from about 8 wt% to about 30wt% of the solvent.
Aspect 38 provides an electrolyte solution of any of Aspects 16-37, wherein a cyclic organic carbonate concentration ranges from about 0 wt% to about 40% wt% of the solvent.
Aspect 39 provides an electrolyte solution of Aspect 38, wherein the cyclic organic carbonate concentration ranges from about 5 wt% to about 25 wt% of the solvent.
Aspect 40 provides an electrolyte solution of any of Aspects 16-39, wherein a linear organic carbonate concentration ranges from 2 wt% to about 95 wt% of the solvent.
Aspect 41 provides an electrolyte solution of Aspect 40, wherein the linear organic carbonate concentration ranges from about 5 wt% to about 60 wt% of the solvent.
Aspect 42 provides an electrolyte solution of any of Aspects 16-41, wherein an additive component concentration ranges from about 0.01 wt% to about 9 wt% of the electrolyte solution.
Aspect 43 provides an electrolyte solution of Aspect 42, wherein the additive component concentration ranges from about 0.5 wt% to about 2 wt% of the electrolyte solution.
Aspect 44 provides an electrolyte solution of any of Aspects 16-43, wherein a total alkali salt concentration is in a range of from about 0.5m to about 1.8m.
Aspect 45 provides an electrolyte solution of Aspect 44, wherein the total alkali salt concentration is in a range of from 0.8m to about 1.4m.
Aspect 46 provides an electrolyte solution of any of Aspects 16-45 wherein the solvent comprises:
   methyl acetate;
   ethylene carbonate;
   dimethyl carbonate.
Aspect 47 provides an electrolyte solution comprising:
   a solvent comprising:
   methyl acetate in a range of from about 0 wt% to about 50 wt% of the solvent;
   ethylene carbonate in a range of from about 0 wt% to about 35 wt% of the solvent;
   dimethyl carbonate in a range of from about 0 wt% to about 25 wt% of the solvent;
additive components comprising from about 0.01 wt% to about 9 wt% of a mixture of compounds each having a structure: 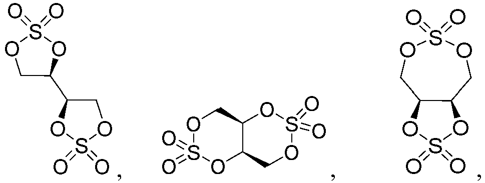 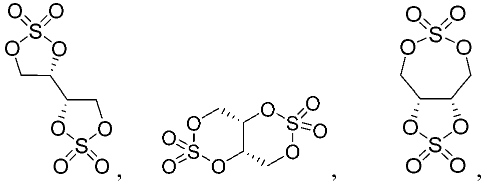 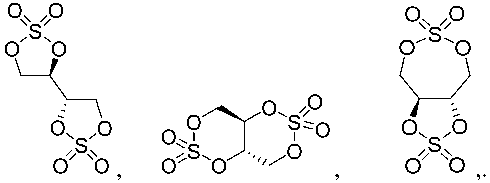 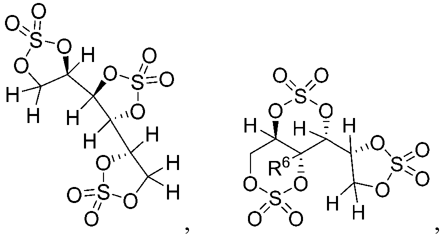 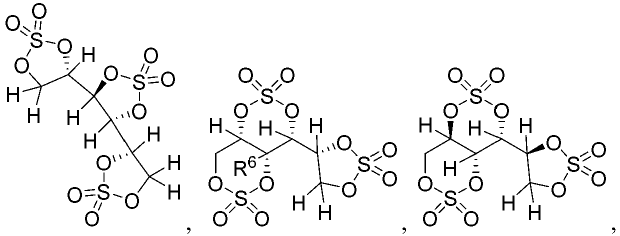 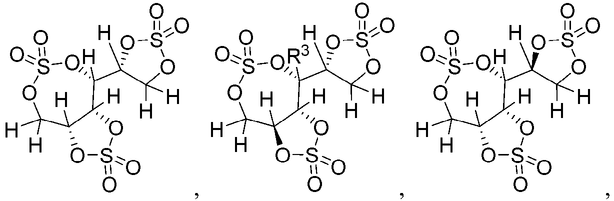 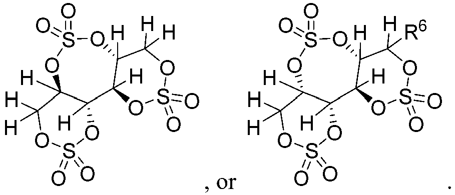 and
   an alkali salt comprising lithium hexafluorophosphate (LiPF₆), lithium bis(fluorosulfonyl)imide (LiFSi), lithium bis-oxalatoborate (LiBOB), lithium difluorooxalatoborate (LiDFOB), lithium bis-trifluoromethansulfonate (LiTFSi), sodium hexafluorophosphate (NaPF₆), sodium bis(fluorosulfonyl)imide (NaFSi), sodium bis-trifluoromethansulfonate (NaTFSi), or a mixture thereof, wherein a concentration of total alkali salt components is in a range of from about 0.6m to about 1.8m.
Aspect 48 provides an energy storage device comprising:
   a cathode;
   an anode;
   a separator between the cathode and the anode; and
   an electrolyte solution comprising:
      a solvent comprising:
      at least one of:
      a linear ester;
      a cyclic organic carbonate;
      a linear organic carbonate; and
      an additive component; and
   an alkali salt.
Aspect 49 provides an energy storage device of Aspect 48, wherein the cathode is selected from a group comprising:
   LiNi_{0.42}Mn_{0.42}Co_{0.16}O₂ (NMC442), LiNi_{0.65}Mn_{0.15}Co_{0.20}O₂ (Ni65),
   LiNi_{0.91}Mn_{0.045}Co_{0.045}O₂ (Ni91), LiNi_{0.63}Mn_{0.31}Co_{0.06}O₂ (NM64), and
   LiNiₓCo_{y}Al_{1-x-y}O₂ where x + y + z = 1 (NCA), and
   LiNiₓMn_{y}Co_{1-x-y}O₂ (NMC) where x + y + z = 1, LiFePO₄ (LFP), LiFeₓMn₁₋ₓPO₄ (LMFP), LiNiₓMn₂₋ₓO₄ (LNMO), LiMn₂O₄ (LMO), and
   NaNiₓFe_{y}Mn_{z}O₂, where x + y + z = 1 (NFM)
Aspect 50 provides an energy storage device of Aspect 48 or 49, wherein an anode is selected from a group comprising:
   graphite; hard carbon; a Si material comprising Si, SiO, LiSiOₓ, SiC or any combination of graphite and Si material; Sn or any combination of hard carbon and Sn, Li₄Ti₅O₁₂ (LTO); Na₂Ti₆O₁₃ (NTO); and NaTi₂(PO₄)₃ (NTP).
Aspect 51 provides an energy storage device of any of Aspects 48-50, wherein the separator comprises a material selected from a group consisting of polyethylene (PE), polypropylene (PP), polyethylene-polypropylene multi-layer (PP/PE/PP), polyvinylidene fluoride (PVDF), anodized alumina (AAO), polyethylene oxide (PEO), polyvinyl alcohol (PVA), β"-alumina, Na₃Zr₂Sl₂PO₁₂ (NASICON), and zeolite A.
Aspect 52 provides an energy storage device of any of Aspects 48-51, wherein at least one of
   the linear ester is selected from a group comprising methyl acetate, ethyl acetate, and methyl propionate;
   the cyclic organic carbonate is selected from a group comprising ethylene carbonate and propylene carbonate, fluoroethylene carbonate, di-fluoroethylene carbonate, and vinylene carbonate; and
   the linear organic carbonate is selected from a group comprising dimethyl carbonate, methyl ethyl carbonate, and diethyl carbonate.
3. Aspect 53 provides an energy storage device of any of Aspects 48-52, wherein the additive component comprises a compound having a structure according to formula (I): 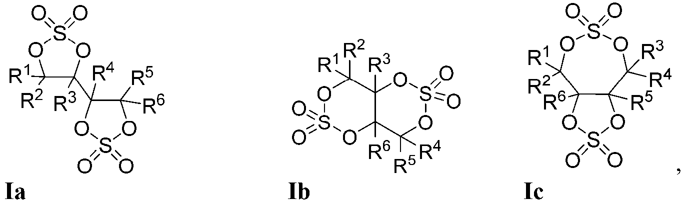 or 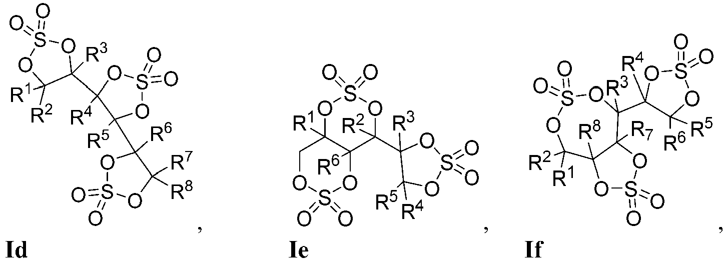 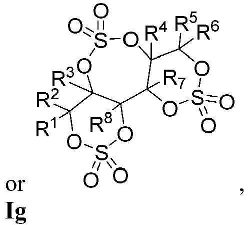 or 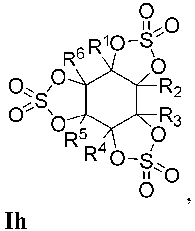
   wherein R¹- R⁸ are each independently H, a halogen, substituted or unsubstituted C₁-C₂₀ hydrocarbyl, or substituted or unsubstituted C₁-C₂₀ haloalkyl;
   or any enantiomer, diastereomer, *meso* compound, or constitutional isomer thereof.
Aspect 54 provides an energy storage device of Aspect 53, wherein the compound has the structure: 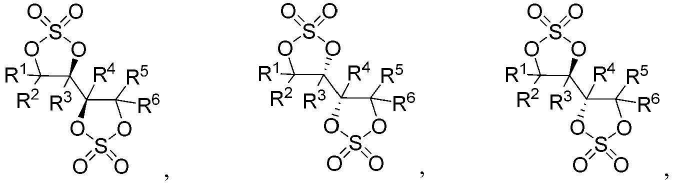 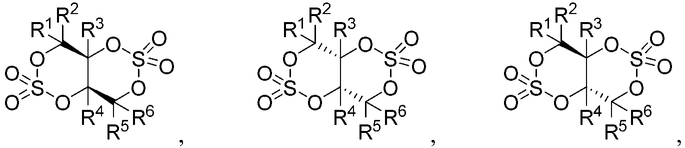 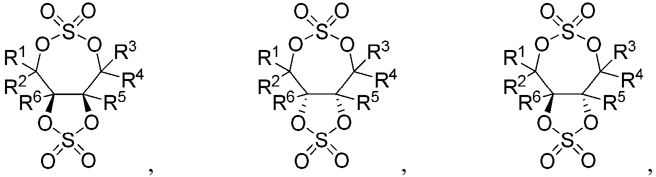 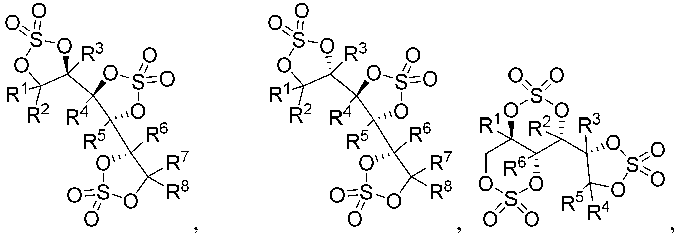 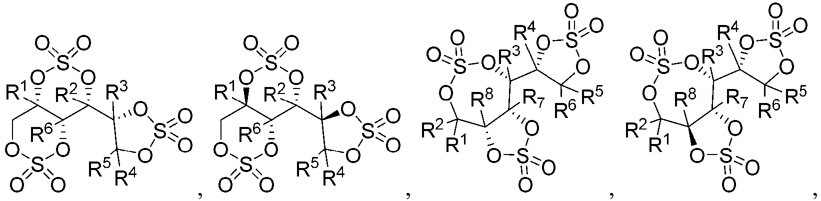 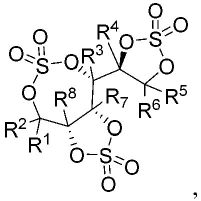 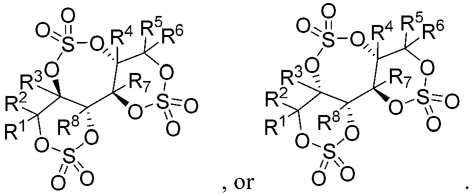
Aspect 55 provides an energy storage device of Aspect 53 or 54, wherein at least one of R¹- R⁸ is H.
Aspect 56 provides an energy storage device of Aspect 53 or 54, wherein at least one of R¹- R⁸ is a halogen.
Aspect 57 provides an energy storage device of Aspect 56, wherein the halogen is F.
Aspect 58 provides an energy storage device of Aspect 53 or 54, wherein at least one of R¹- R⁸ is substituted or unsubstituted C₁-C₂₀ alkyl.
Aspect 59 provides an energy storage device of Aspect 53 or 54, wherein at least one of R¹- R⁸ is substituted or unsubstituted C₁-C₂₀ aryl.
Aspect 60 provides an energy storage device of Aspect 53 or 54, wherein at least one of R¹- R⁸ are substituted or unsubstituted C₁-C₂₀ haloalkyl.
Aspect 61 provides an energy storage device of Aspect 60, wherein at least one of R¹- R^{8 a}re substituted or unsubstituted C₁-C₂₀ fluoroalkyl.
Aspect 62 provides an energy storage device of Aspect 61 comprising a compound having the structure: 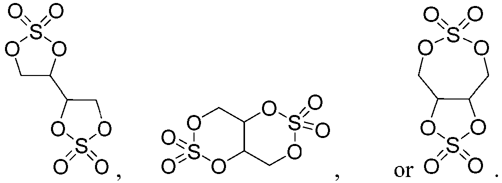
Aspect 63 provides an energy storage device of Aspect 62 comprising a compound having the structure: 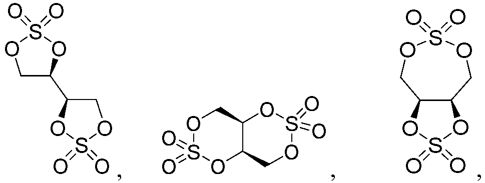 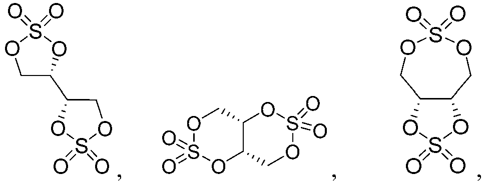 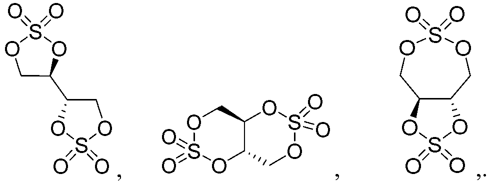 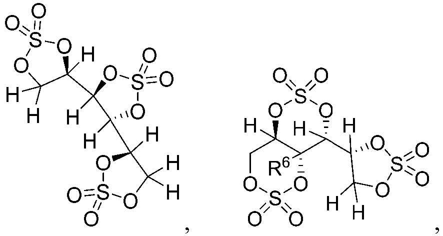 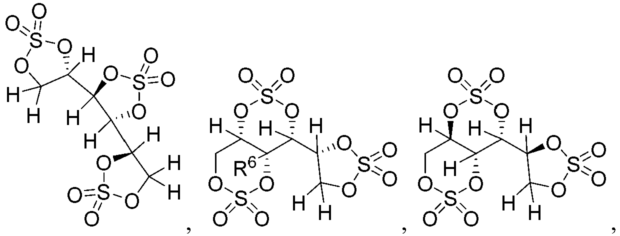 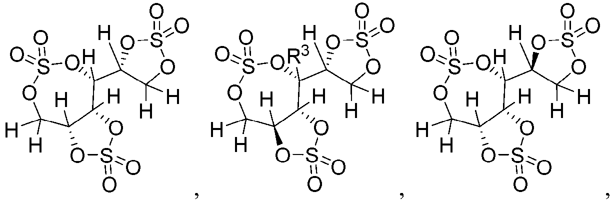 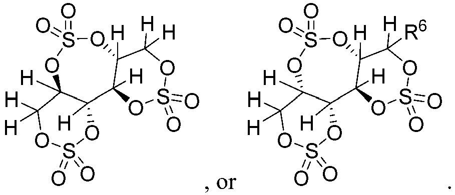
Aspect 64 provides an energy storage device of any of Aspects 48-63, wherein the additive component comprises a mixture of compounds each having a structure according to formula (I): 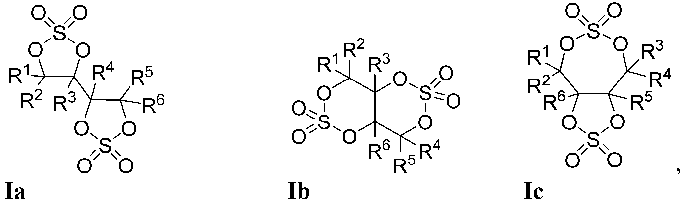 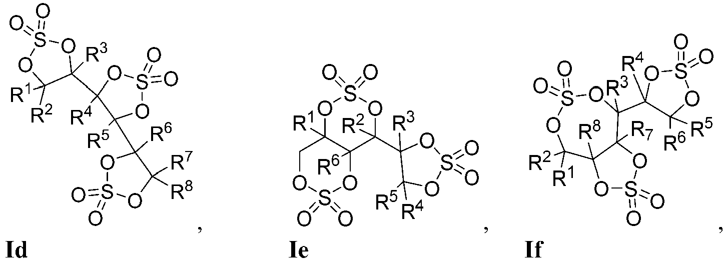 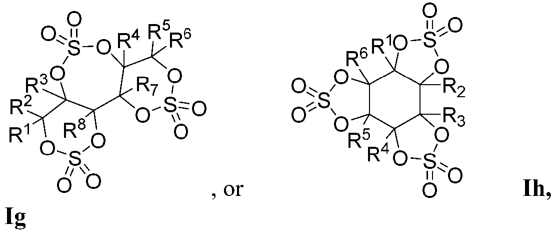 wherein R¹- R⁸ are each independently H, a halogen, substituted or unsubstituted C₁-C₂₀ hydrocarbyl, or substituted or unsubstituted C₁-C₂₀ haloalkyl,
   or any enantiomer, diastereomer, *meso* compound, or constitutional isomer thereof.
Aspect 65 provides an energy storage device of Aspect 64, wherein the additive component comprises a mixture of compounds each having the structure: 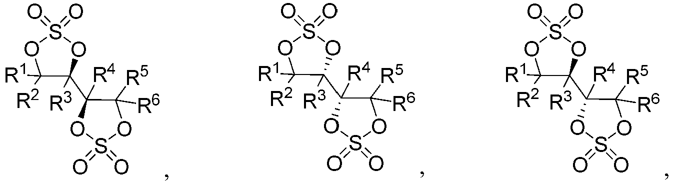 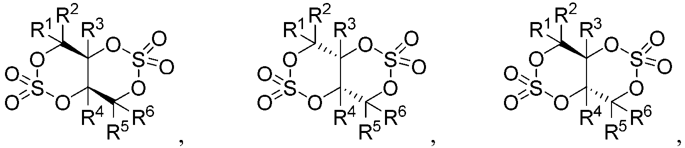 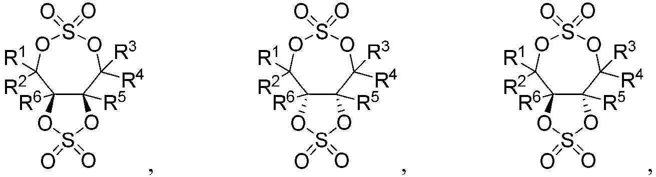 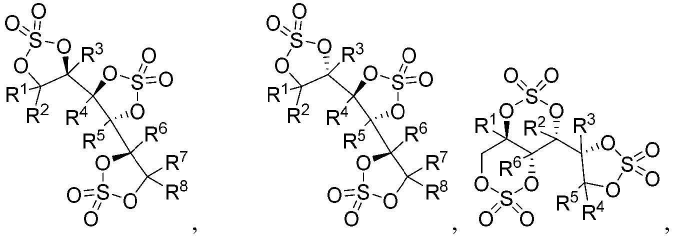 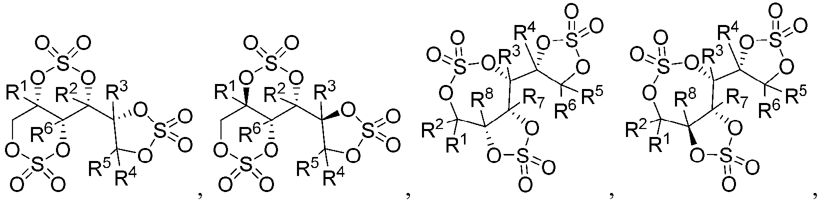 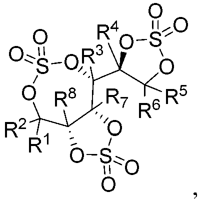 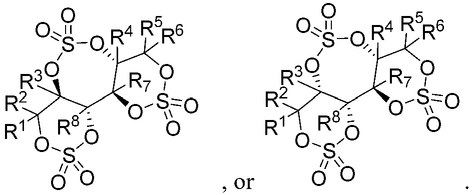
Aspect 66 provides an energy storage device of Aspect 65 wherein the additive component comprises a mixture of compounds each having the structure: 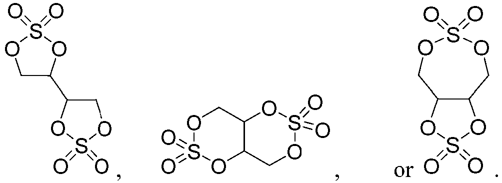
Aspect 67 provides an energy storage device of Aspect 66, wherein the additive component comprises a mixture of compounds each having the structure: 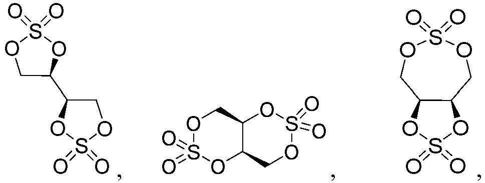 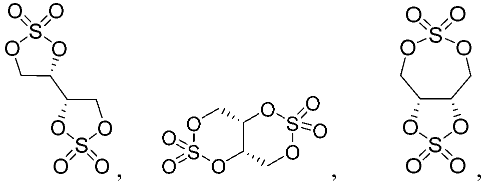 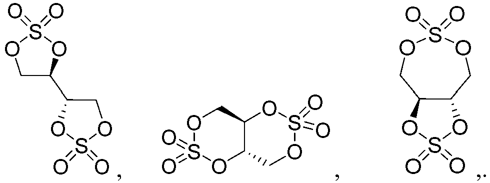 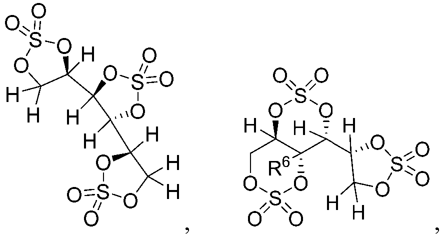 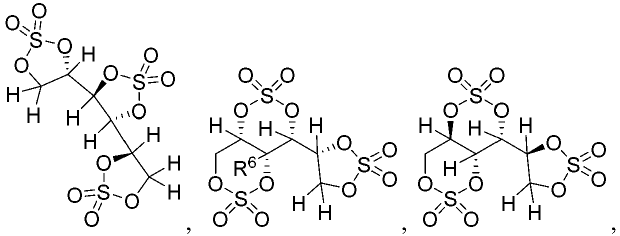 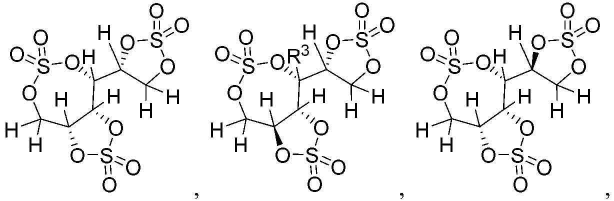 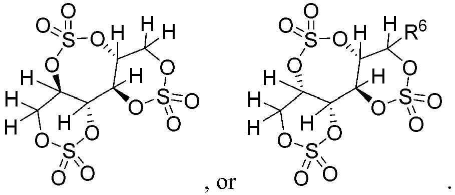
Aspect 68 provides an energy storage device of any of Aspects 48-67, wherein the alkali salt comprises lithium hexafluorophosphate (LiPF₆), lithium bis(fluorosulfonyl)imide (LiFSi), lithium bis-oxalatoborate (LiBOB), lithium difluorooxalatoborate (LiDFOB), lithium bis-trifluoromethansulfonate (LiTFSi), sodium hexafluorophosphate (NaPF₆), sodium bis(fluorosulfonyl)imide (NaFSi), sodium bis-trifluoromethansulfonate (NaTFSi), or a mixture thereof.
Aspect 69 provides an energy storage device of any of Aspects 48-68, wherein a linear ester concentration is in a range of from about 0 wt% to about 80 wt% of the solvent.
Aspect 70 provides an energy storage device of Aspect 69, wherein the linear ester concentration is in a range of from about 5 wt% to about 50 wt% of the solvent.
Aspect 71 provides an energy storage device of Aspect 70, wherein the linear ester concentration is in a range of from about 8 wt% to about 30 wt% of the solvent.
Aspect 72 provides an energy storage device of any of Aspects 48-71, wherein the cyclic organic carbonate is in a range of from about 0 wt% to about 40 wt% of the solvent.
Aspect 73 provides an energy storage device of any of Aspect 72, wherein the cyclic organic carbonate is in a range of from about 5 wt% to about 25 wt% of the solvent.
Aspect 74 provides an energy storage device of any of Aspects 48-73, wherein a linear organic carbonate concentration ranges from 2 wt% to about 95 wt% of the solvent.
Aspect 75 provides an energy storage device of Aspect 74, wherein the linear organic carbonate ranges from about 5 wt% to about 60 wt% of the solvent.
Aspect 76 provides an energy storage device of any of Aspects 48-75, wherein the additive component ranges from about 0.01 wt% to about 9 wt% of the electrolyte solution.
Aspect 77 provides an energy storage device of Aspect 76, wherein the additive component ranges from about 0.5 wt% to about 2 wt% of the electrolyte solution.
Aspect 78 provides an energy storage device of any of Aspects 48-77, wherein an alkali salt concentration is in a range of from about 0.6m to about 1.8m.
Aspect 79 provides an energy storage device of Aspect 78, wherein the alkali salt concentration is in a range of from about 0.8m to about 1.4m.
Aspect 80 provides an energy storage device comprising:
   a cathode;
   an anode;
   a separator between the cathode and the anode; and
   an electrolyte solution comprising:
      a solvent comprising:
         at least one of:
         methyl acetate;
         ethylene carbonate;
         dimethyl carbonate, and
         an additive component; and
      an alkali salt.
Aspect 81 provides an energy storage device of any of Aspects 48-80, having an operating temperature of between about -40 to about 90°C.
Aspect 82 provides an energy storage device of Aspect 81, wherein the operating temperature is between about -30 to about 75°C.
Aspect 83 provides an energy storage device of any of Aspects 48 to 82 for use in portable applications, vehicles, domestic buildings, commercial building, or grid energy storage.
Aspect 84 provides a method of fabricating an energy storage device, the method comprising:
   placing a separator between a cathode and an anode;
   inserting the cathode, the anode, and the separator into a housing;
   adding an electrolyte solution to the housing;
   and contacting the electrolyte solution with the cathode and the anode,
   wherein the electrolyte solution comprises:
      a solvent comprising:
         at least one of:
         linear ester,
         a cyclic organic carbonate,
         linear carbonate, and
         one or more additive components, and
      an alkali salt.
Aspect 85 provides a method of Aspect 84, wherein the separator comprises a material selected from a group consisting of polyethylene (PE), polypropylene (PP), polyethylene-polypropylene multi-layer (PP/PE/PP), polyvinylidene fluoride (PVDF), anodized alumina (AAO), polyethylene oxide (PEO), polyvinyl alcohol (PVA), β"-alumina, Na₃Zr₂Si₂PO₁₂ (NASICON), and zeolite A.
Aspect 86 provides a method of Aspect 84 or 85, wherein a cathode is selected from a group comprising:
   LiNi_{0.42}Mn_{0.42}Co_{0.16}O₂ (NMC442), LiNi_{0.65}Mn_{0.15}Co_{0.20}O₂ (Ni65), LiNi_{0.91}Mn_{0.045}Co_{0.045}O₂ (Ni91), LiNi_{0.63}Mn_{0.31}Co_{0.06}O₂ (NM64), and LiNiₓCo_{y}Al_{1-x-y}O₂ where x + y + z = 1 (NCA), and
   LiNiₓMn_{y}Co_{1-x-y}O₂ (NMC) where x + y + z = 1, LiFePO₄ (LFP), LiFeₓMn₁₋ₓPO₄ (LMFP), LiNiₓMn₂₋ₓO₄ (LNMO), and LiMn₂O₄ (LMO), where x + y + z = 1 (NFM).
Aspect 87 provides a method of any of Aspects 84-86, wherein an anode is selected from a group comprising: graphite, hard carbon, Si material (Si, SiO, LiSiOₓ, SiC) or any combination of graphite and Si material, Sn or any combination of hard carbon and Sn,
   Li₄Ti₅O₁₂ (LTO), Na₂Ti₆O₁₃ (NTO), and NaTi₂(PO₄)₃ (NTP).
Aspect 88 provides a method of any of Aspects 84-86, wherein the electrolyte solution comprises at least one of:
   the linear ester is selected from a group comprising methyl acetate, ethyl acetate, and methyl propionate;
   the cyclic organic carbonate is selected from a group comprising ethylene carbonate and propylene carbonate; fluoroethylene carbonate, di-fluoroethylene carbonate, and vinylene carbonate; and
   the linear carbonate is selected from a group comprising dimethyl carbonate, methyl ethyl carbonate, and diethyl carbonate.
Aspect 89 provides a method of any of Aspects 84-88, wherein the electrolyte solution comprises an alkali salt selected from a group comprising lithium hexafluorophosphate (LiPF₆), lithium bis(fluorosulfonyl)imide (LiFSi), lithium bis-oxalatoborate (LiBOB), lithium difluorooxalatoborate (LiDFOB), lithium bis-trifluoromethansulfonate (LiTFSi) or a mixture thereof.
4. Aspect 90 provides a method of any of Aspects 84-89, wherein an additive component comprises a compound having a structure according to formula (I): 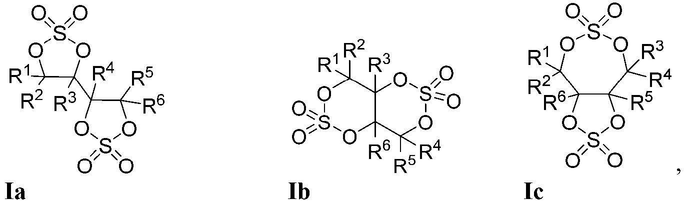 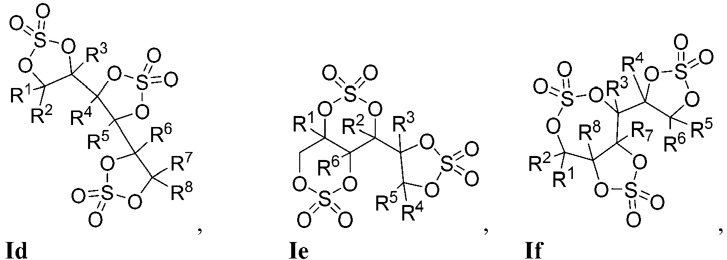 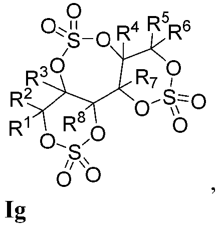 or 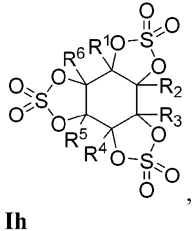 wherein R¹- R⁸ are each independently H, a halogen, substituted or unsubstituted C₁-C₂₀ hydrocarbyl, or substituted or unsubstituted C₁-C₂₀ haloalkyl,
   or any enantiomer, diastereomer, *meso* compound, or constitutional isomer thereof.
Aspect 91 provides a method of Aspect 90, wherein an additive component comprises the compound having the structure: 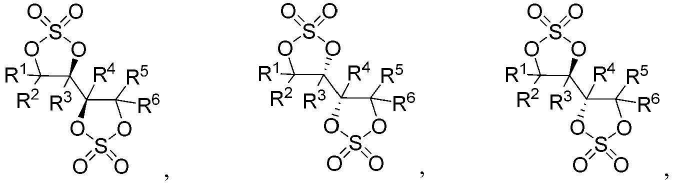 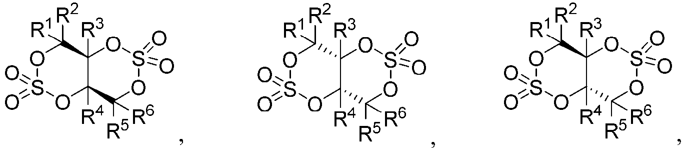 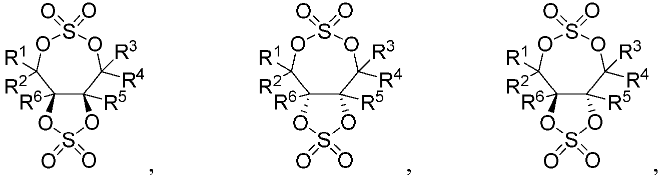 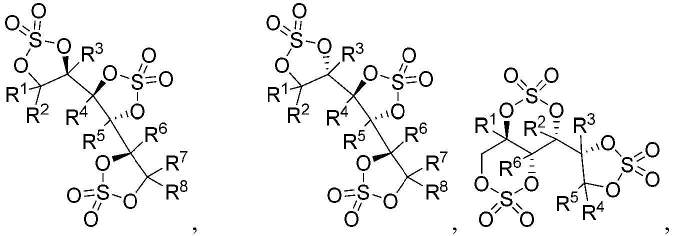 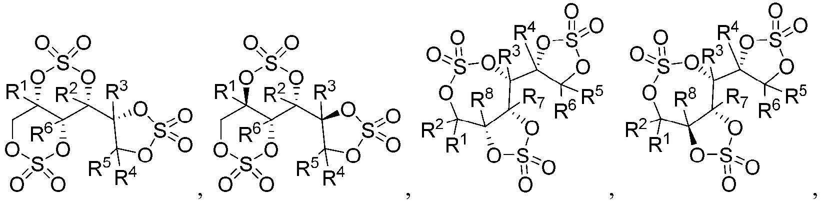 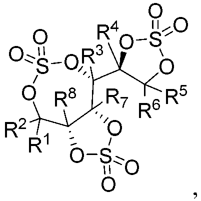 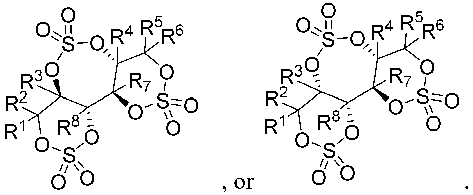
Aspect 92 provides a method of Aspects 90 or 91, wherein at least one of R¹- R⁸ is H.
Aspect 93 provides a method of Aspects 90 or 91, wherein at least one of R¹- R⁸ is a halogen.
Aspect 94 provides a method of Aspect 93, wherein the halogen is F.
Aspect 95 provides a method of Aspect 90 or 91, wherein at least one of R¹- R⁸ is substituted or unsubstituted C₁-C₂₀ alkyl.
Aspect 96 provides a method of Aspect 90 or 91, wherein at least one of R¹- R⁸ is substituted or unsubstituted C₁-C₂₀ aryl.
Aspect 97 provides a method of Aspect 90 or 91, wherein at least one of R¹- R⁸ is substituted or unsubstituted C₁-C₂₀ haloalkyl.
Aspect 98 provides a method of Aspect 97, wherein at least one of R¹- R⁸ is substituted or unsubstituted C₁-C₂₀ fluoroalkyl.
Aspect 99 provides a method of Aspect 92 wherein an additive component comprises the compound having the structure: 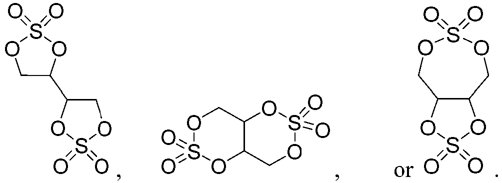
Aspect 100 provides a method of Aspect 93 wherein an additive component comprises the compound having the structure: 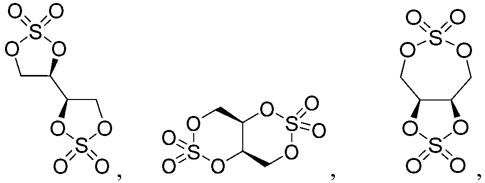 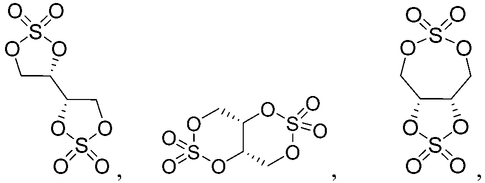 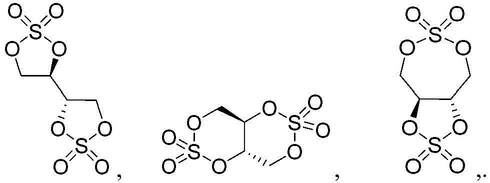 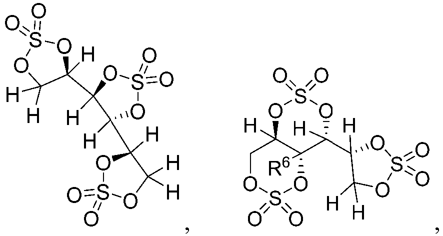 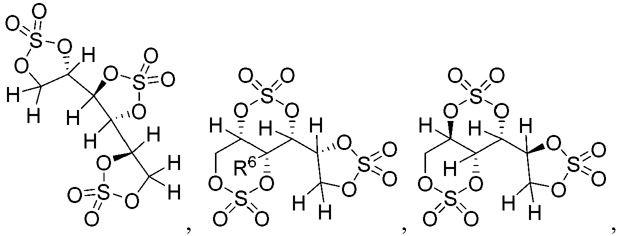 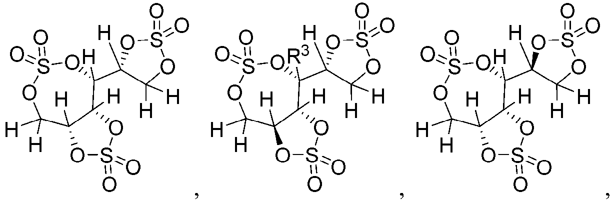 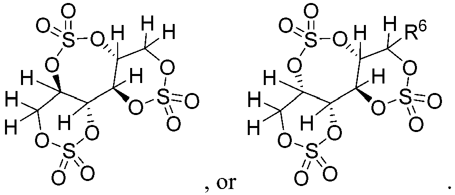
Aspect 101 provides a method of any of Aspects 84-100, wherein an additive component comprises a mixture of compounds each having a structure according to formula (I): 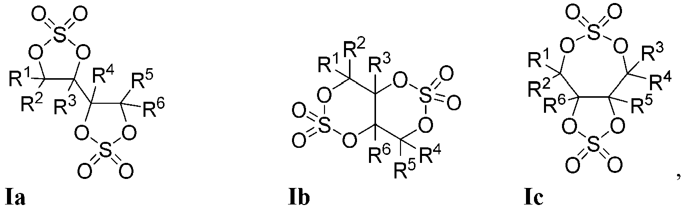 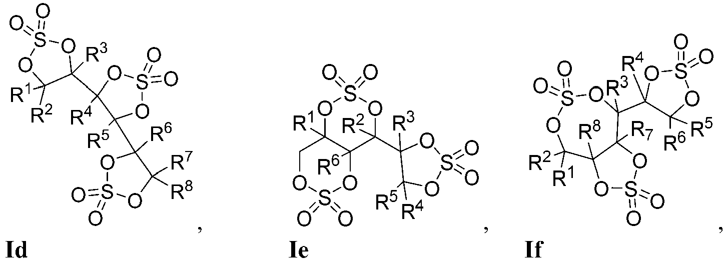 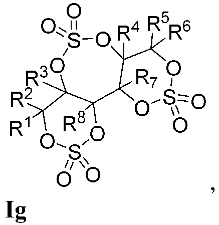 or 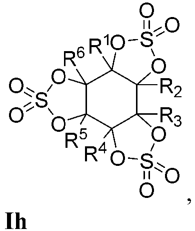
   wherein R¹- R⁸ are each independently H, a halogen, substituted or unsubstituted C₁-C₂₀ hydrocarbyl, or substituted or unsubstituted C₁-C₂₀ haloalkyl,
   or any enantiomer, diastereomer, *meso* compound, or constitutional isomer thereof.
Aspect 102 provides a method of Aspect 101, wherein the additive component comprises the mixture of compounds each having the structure: 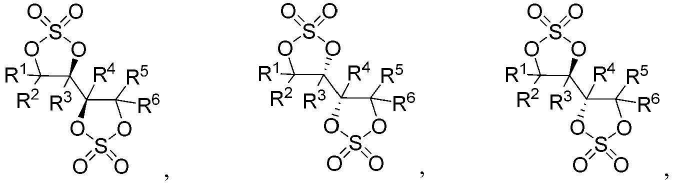 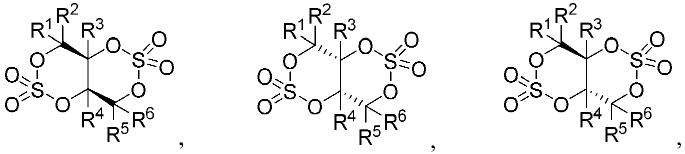 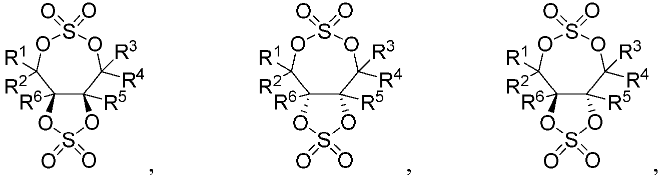 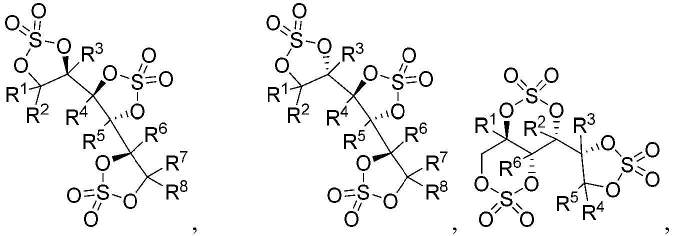 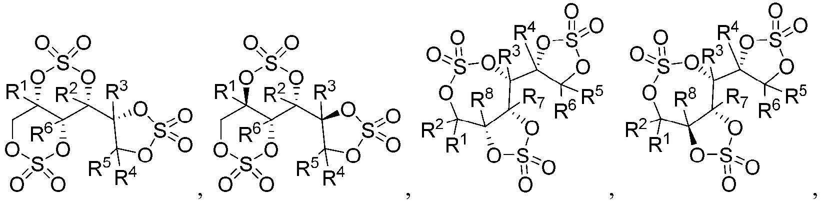 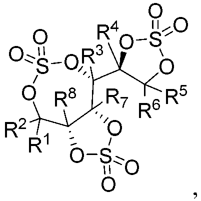 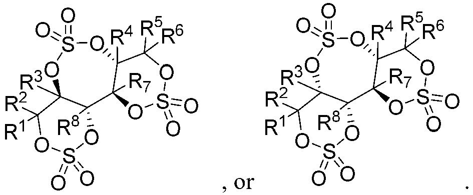
Aspect 103 provides a method of Aspect 102 wherein the additive component comprises the mixture of compounds each having the structure: 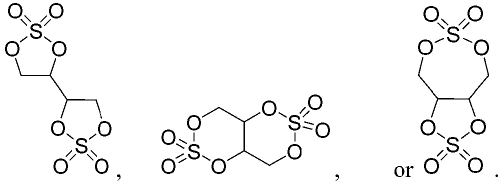
Aspect 104 provides a method of Aspect 103, wherein the additive component comprises a mixture of compounds each having the structure: 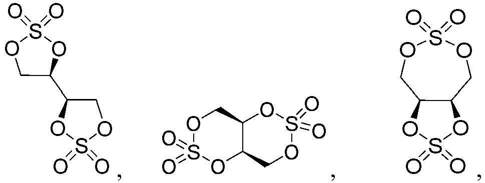 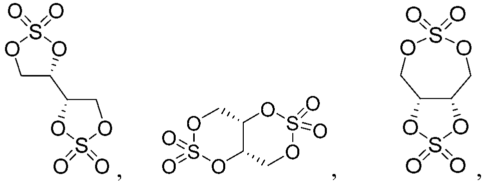 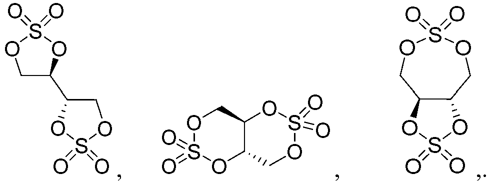 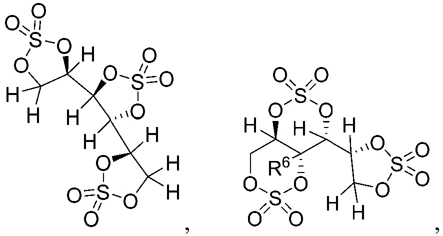 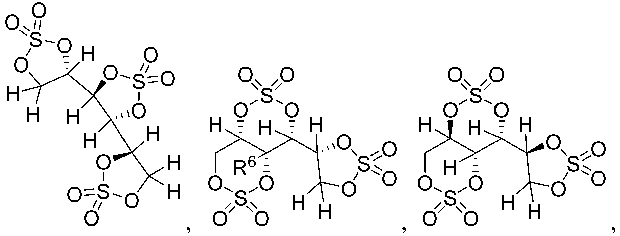 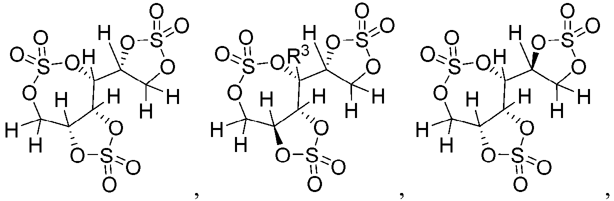 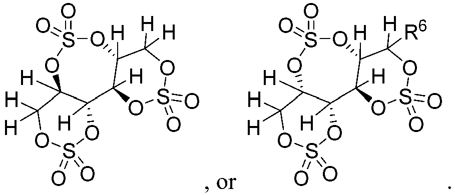
Aspect 105 provides a method of any of Aspects 84-104, wherein a linear ester concentration is in a range of from about 0 wt% to about 80 wt% of the solvent.
Aspect 106 provides a method of Aspect 105, wherein the linear ester concentration is in a range of from about 5 wt% to about 50 wt% of the solvent.
Aspect 107 provides a method of Aspect 106, wherein the linear ester concentration is in a range of from about 8 wt% to about 30 wt% of the solvent.
Aspect 108 provides a method of any of Aspects 84-107, wherein a cyclic organic carbonate concentration is in a range of from about 0 wt% to about 40 wt% of the solvent.
Aspect 109 provides a method of Aspect 108, wherein the cyclic organic carbonate concentration is in a range of from about 5 wt% to about 25 wt% of the solvent.
Aspect 110 provides a method of any of Aspects 84-109, wherein a linear organic carbonate concentration ranges from 2 wt% to about 95 wt% of the solvent.
Aspect 111 provides a method of any of Aspects 110, wherein the linear organic carbonate concentration ranges from about 5 wt% to about 60 wt% of the solvent.
Aspect 112 provides a method of any of Aspects 84-111, wherein an additive component concentration ranges from about 0.01 wt% to about 9 wt% of the electrolyte solution.
Aspect 113 provides a method of Aspect 112, wherein the additive component concentration ranges from about 0.5 wt% to about 2 wt% of the electrolyte solution.
Aspect 114 provides a method of Aspects 84-113, wherein an alkali salt concentration is in a range of from about 0.8m to about 1.4m.
Aspect 115 provides a method of Aspect 114, wherein the alkali salt concentration is in a range of from about 0.6m to about 1.8m.
Aspect 116 provides a vehicle, comprising:
   a motor to provide propulsion power to the vehicle; and
   an energy storage device coupled to the motor to provide energy to the motor, wherein the energy storage device comprises:
      a cathode;
      an anode;
      a separator between the cathode and the anode; and
      an electrolyte solution comprising:
         a solvent comprising:
         at least one of:
         a linear ester;
         a cyclic organic carbonate;
         a linear organic carbonate; and
         an additive component; and
      an alkali salt.
Aspect 117 provides a vehicle of any of Aspect 116, wherein the cathode is selected from a group comprising:
   LiNi_{0.42}Mn_{0.42}Co_{0.16}O₂ (NMC442), LiNi_{0.65}Mn_{0.15}Co_{0.20}O₂ (Ni65), LiNi_{0.91}Mn_{0.045}Co_{0.045}O₂ (Ni91), LiNi_{0.63}Mn_{0.31}Co_{0.06}O₂ (NM64), and
   LiNiₓCo_{y}Al_{1-x-y}O₂ where x + y + z = 1 (NCA), and LiNiₓMn_{y}Co_{1-x-y}O₂ (NMC) where x + y + z = 1, LiFePO₄ (LFP), LiFeₓMn₁₋ₓPO₄ (LMFP), LiNiₓMn₂₋ₓO₄ (LNMO), LiMn₂O₄ (LMO), and NaNiₓFe_{y}Mn_{z}O₂, where x + y + z = 1 (NFM).
Aspect 118 provides a vehicle of Aspect 116 or 117, wherein the anode is selected from a group comprising: graphite; hard carbon; a Si material comprising Si, SiO, LiSiOₓ, SiC or any combination of graphite and Si material; Sn or any combination of hard carbon and Sn; Li₄Ti₅O₁₂ (LTO); Na₂Ti₆O₁₃ (NTO); and NaTi₂(PO₄)₃ (NTP).
Aspect 119 provides a vehicle of any of Aspects 116-118, wherein the separator comprises a material selected from a group consisting of polyethylene (PE), polypropylene (PP), polyethylene-polypropylene multi-layer (PP/PE/PP), polyvinylidene fluoride (PVDF), anodized alumina (AAO), polyethylene oxide (PEO), polyvinyl alcohol (PVA), β"-alumina, Na₃Zr₂Si₂PO₁₂ (NASICON), and zeolite A.
Aspect 120 provides a vehicle of any of Aspects 116-119, wherein an alkali salt component is selected from a group comprising lithium hexafluorophosphate (LiPF₆), lithium bis(fluorosulfonyl)imide (LiFSi), lithium bis-oxalatoborate (LiBOB), lithium difluorooxalatoborate (LiDFOB), lithium bis-trifluoromethansulfonate (LiTFSi), sodium hexafluorophosphate (NaPF₆), sodium bis(fluorosulfonyl)imide (NaFSi), sodium bis-trifluoromethansulfonate (NaTFSi), or a mixture thereof.
Aspect 121 provides a vehicle of any of Aspects 116-120, wherein at least one of
   the linear ester is selected from a group comprising methyl acetate, ethyl acetate, and methyl propionate,
   the cyclic organic carbonate is selected from a group comprising ethylene carbonate and propylene carbonate; and
   the linear organic carbonate is selected from a group comprising dimethyl carbonate, methyl ethyl carbonate, and diethyl carbonate.
Aspect 122 provides a vehicle of any of Aspects 116-121, wherein the
   additive component comprises a compound having a structure according to formula (I): 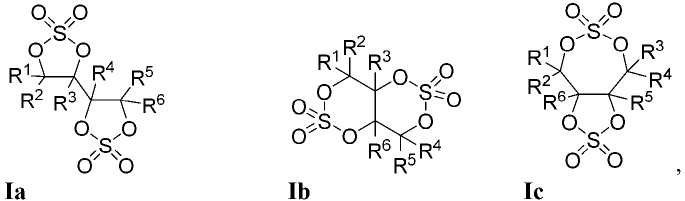 or 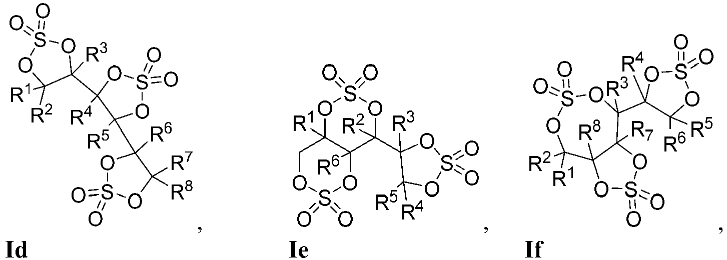 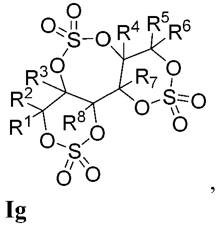 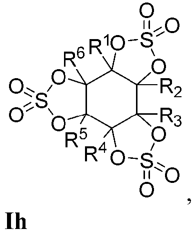
   wherein R¹- R⁸ are each independently H, a halogen, substituted or unsubstituted C₁-C₂₀ hydrocarbyl, or substituted or unsubstituted C₁-C₂₀ haloalkyl,
   or any enantiomer, diastereomer, *meso* compound, or constitutional isomer thereof.
Aspect 123 provides a vehicle of Aspect 122, wherein the compound has the structure: 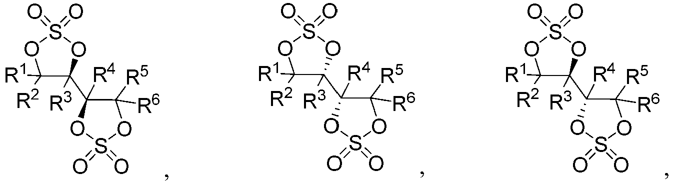 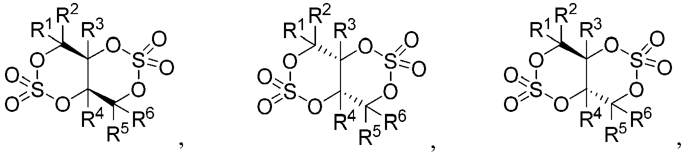 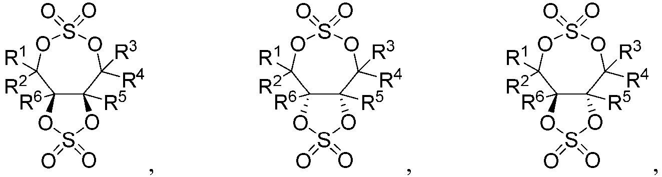 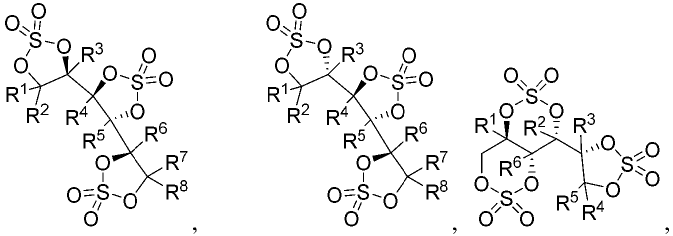 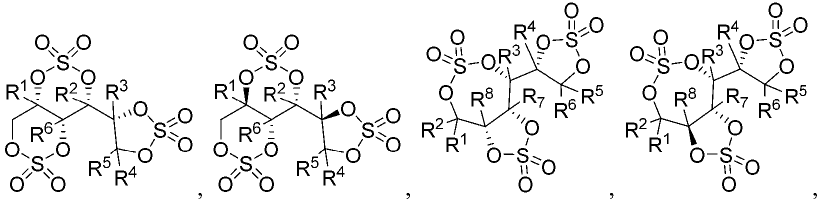 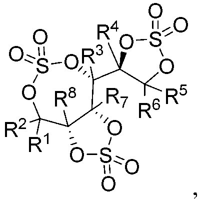 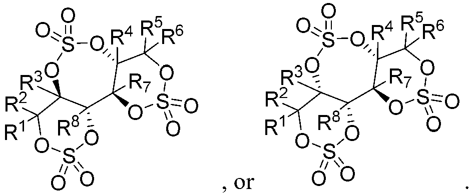
Aspect 124 provides a vehicle of Aspect 122 or 123, wherein at least one of R¹- R⁸ is H.
Aspect 125 provides a vehicle of Aspect 122 or 123, wherein at least one of R¹- R⁸ is a halogen.
Aspect 126 provides a vehicle of Aspect 125, wherein the halogen is F.
Aspect 127 provides a vehicle of Aspect 122 or 123, wherein at least one of R¹- R⁸ is substituted or unsubstituted C₁-C₂₀ alkyl.
Aspect 128 provides a vehicle of Aspect 122 or 123, wherein at least one of R¹- R⁸ is substituted or unsubstituted C₁-C₂₀ aryl.
Aspect 129 provides a vehicle of Aspect 122 or 123, wherein at least one of R¹- R⁸ is substituted or unsubstituted C₁-C₂₀ haloalkyl.
Aspect 130 provides a vehicle of Aspect 129, wherein at least one of R¹- R⁸ is substituted or unsubstituted C₁-C₂₀ fluoroalkyl.
Aspect 131 provides a vehicle of Aspect 129 comprising a compound having the structure: 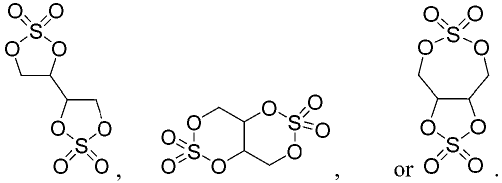
Aspect 132 provides a vehicle of Aspect 131 comprising a compound having the structure: 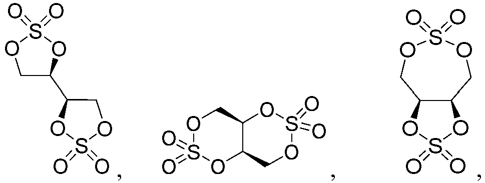 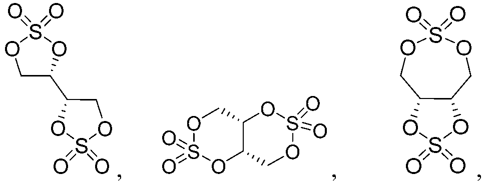 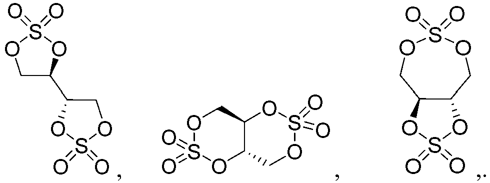 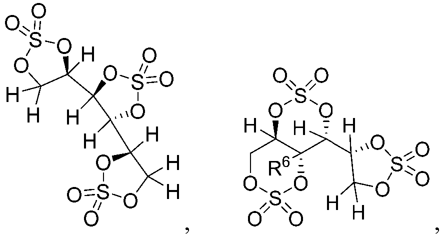 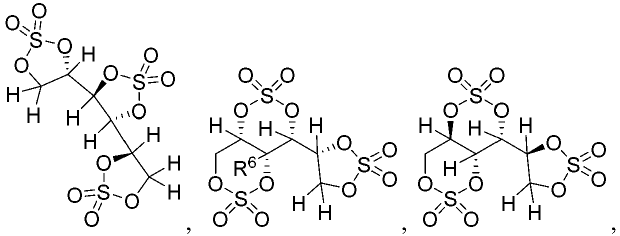 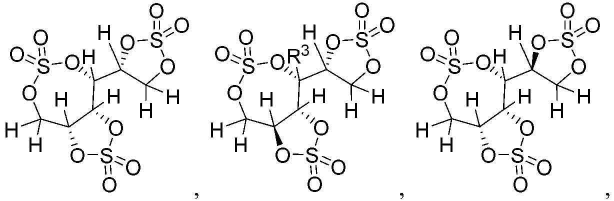 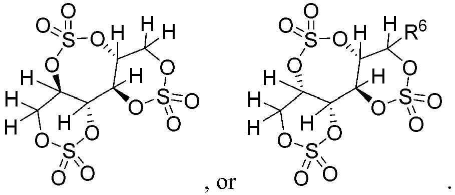
Aspect 133 provides a vehicle of any of Aspects 116-132, wherein the additive component comprises a mixture of compounds each having a structure according to formula (I): 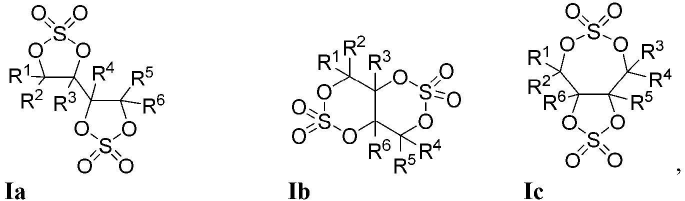 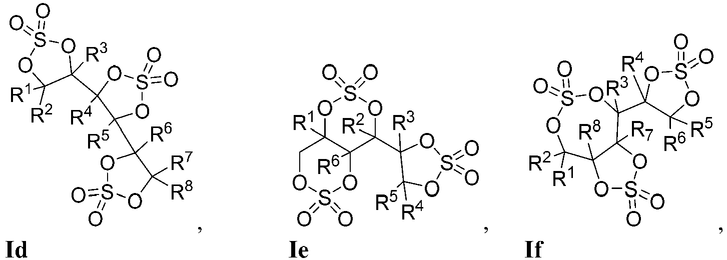 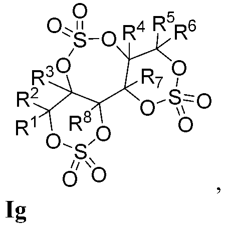 or 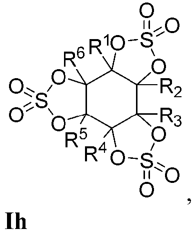
   wherein R¹- R⁸ are each independently H, a halogen, substituted or unsubstituted C₁-C₂₀ hydrocarbyl, or substituted or unsubstituted C₁-C₂₀ haloalkyl,
   or any enantiomer, diastereomer, *meso* compound, or constitutional isomer thereof..
Aspect 134 provides a vehicle of Aspect 133, wherein the additive component comprises a mixture of compounds each having the structure: 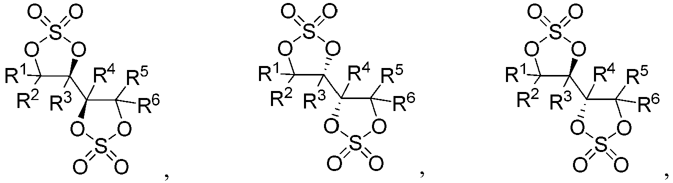 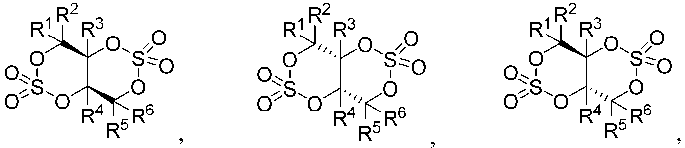 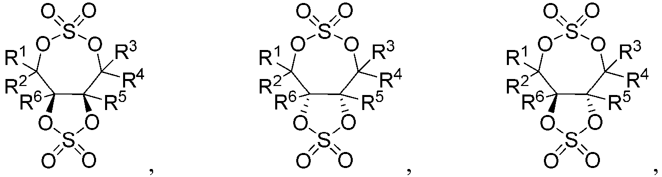 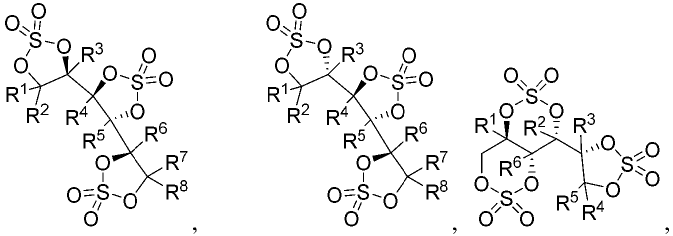 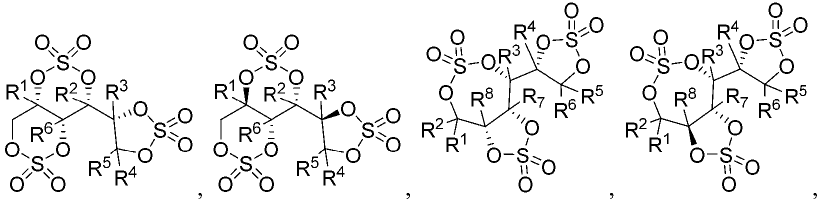 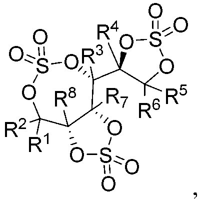 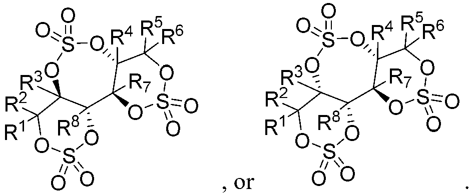
Aspect 135 provides a vehicle of Aspect 134, wherein the additive component comprises a mixture of compounds each having the structure: 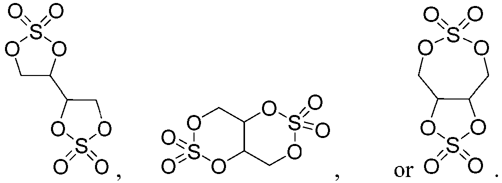
Aspect 136 provides a vehicle of Aspect 135, wherein the additive component comprises a mixture of compounds each having the structure: 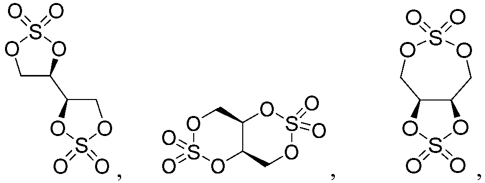 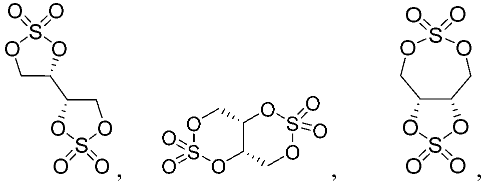 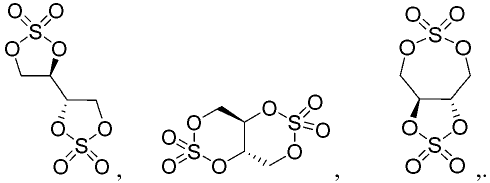 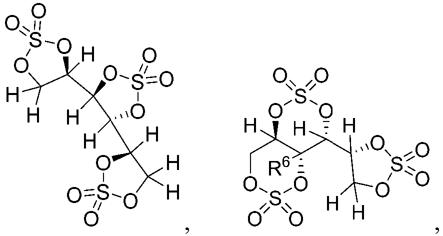 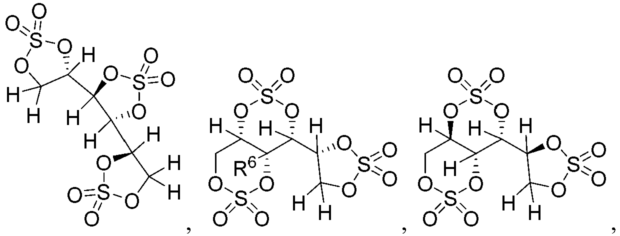 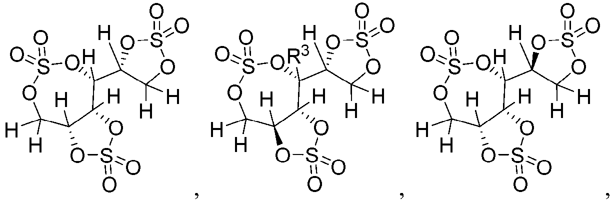 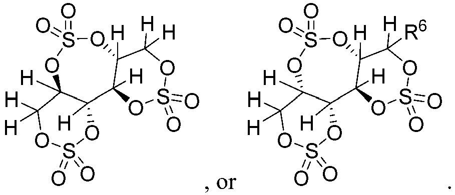
Aspect 137 provides a vehicle of any of Aspects 116-136, wherein a linear ester concentration is in a range of from about 0 wt% to about 80 wt% of the solvent.
Aspect 138 provides a vehicle of Aspect 137, wherein the linear ester concentration is in a range of from about 5 wt% to about 50 wt% of the solvent.
Aspect 139 provides a vehicle of Aspect 138, wherein the linear ester concentration is in a range of from about 8 wt% to about 30 wt% of the solvent.
Aspect 140 provides a vehicle of any of Aspects 116-139, wherein a cyclic organic carbonate concentration is in a range of from about 0 wt% to about 40 wt% of the solvent.
Aspect 141 provides a vehicle of Aspect 140, wherein the cyclic organic carbonate concentration is in a range of from about 5 wt% to about 25 wt% of the solvent.
Aspect 142 provides a vehicle of any of Aspects 116-141, wherein a linear organic carbonate concentration ranges from 2 wt% to about 95 wt% of the solvent.
Aspect 143 provides a vehicle of Aspect 142, wherein the linear organic carbonate concentration ranges from about 5 wt% to about 60 wt% of the solvent.
Aspect 144 provides a vehicle of any of Aspects 116-143, wherein an additive component concentration ranges from about 0.01 wt% to about 9 wt% of the electrolyte solution.
Aspect 145 provides a vehicle of Aspect 144, wherein the additive component concentration ranges from about 0.5 wt% to about 2 wt% of the electrolyte solution.
Aspect 146 provides a vehicle of any of Aspects 116-145, wherein an alkali salt concentration is in a range of from about 0.6m to about 1.8m.
Aspect 147 provides a vehicle of Aspect 146, wherein the alkali salt concentration is in a range of from about 0.8m to about 1.4m.
Aspect 148 provides a vehicle of any of Aspects 116-147 having an operating temperature between about -30 to about 60°C.
Aspect 149 provides a vehicle of Aspect 148, wherein the operating temperature is between about -20 to about 45°C.
Aspect 150 provides a vehicle of any of Aspects 116 to 148, wherein the vehicle is a car, passenger vehicle, truck, bus, shuttle, motorcycle, scooter, utility vehicle, construction vehicle, mining vehicle, agricultural vehicle, aircraft, plane, helicopter, vertical takeoff and landing (eVTOL) vehicle, drone, unmanned aerial vehicle (UAV), airship, space vehicle, boat, ship, ferry, personal watercraft, submersible, marine vessel, port vehicle, train, tram, light rail vehicle, or subway car.

## Claims

1. A compound having a structure according to formula (I): 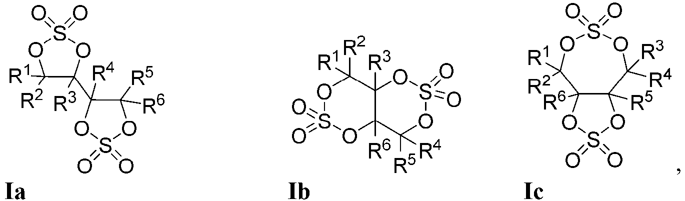 or 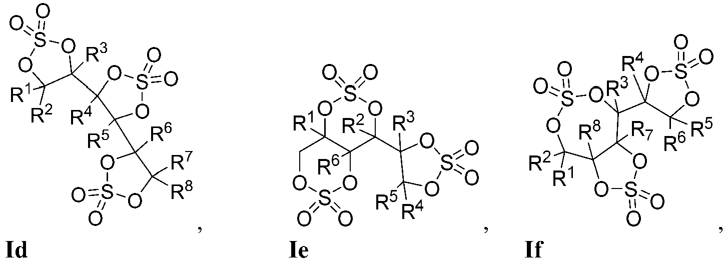 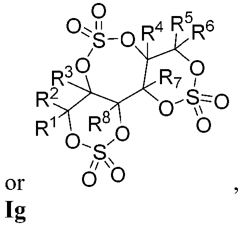 or 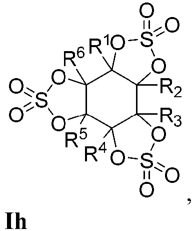
wherein R¹- R⁸ are each independently H, a halogen, substituted or
unsubstituted C₁-C₂₀ hydrocarbyl, or substituted or unsubstituted C₁-C₂₀ haloalkyl,
or any enantiomer, diastereomer, *meso* compound, or constitutional isomer thereof.

2. The compound of claim 1, having the structure:

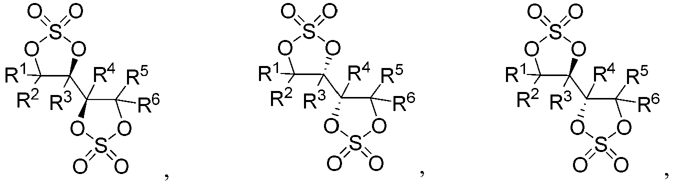

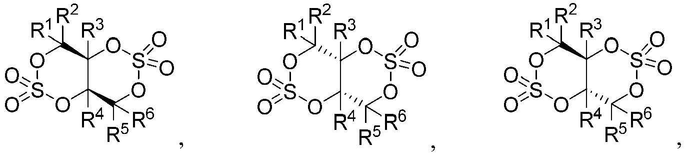

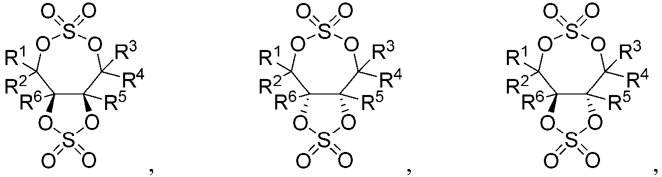

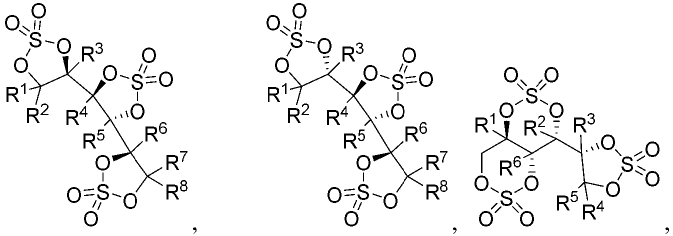

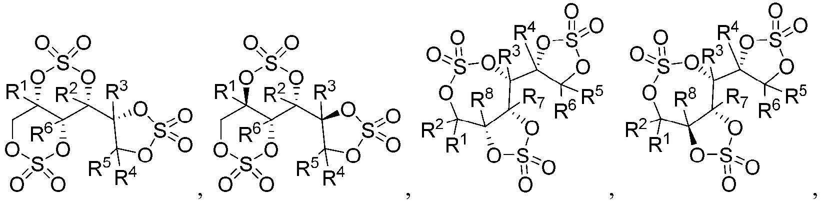

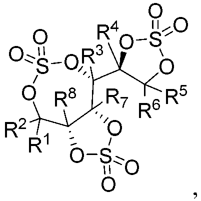

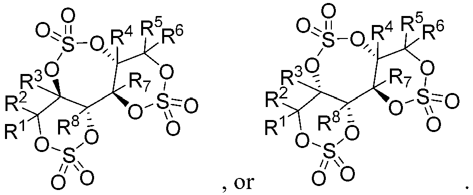

3. The compound of claim 1 or 2, wherein at least one of R¹- R⁸ is H and/or wherein at least one of R¹- R⁸ is a halogen, optionally wherein the halogen is F.

4. The compound of any preceding claim, wherein at least one of R¹- R⁸ is substituted or unsubstituted C₁-C₂₀ alkyl and/or wherein at least one of R¹-R⁸ is substituted or unsubstituted C₁-C₂₀ aryl.

5. The compound of any preceding claim, wherein at least one of R¹- R⁸ is substituted or unsubstituted C₁-C₂₀ haloalkyl, optionally wherein at least one of R¹- R⁸ is substituted or unsubstituted C₁-C₂₀ fluoroalkyl.

6. The compound of any preceding claim, wherein the compound has the structure:

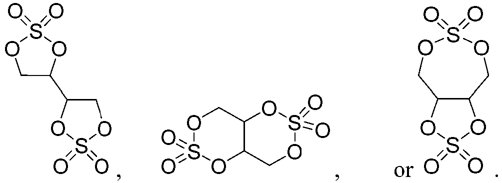

7. An electrolyte solution comprising:
a solvent comprising:
at least one of:
a linear ester;
a cyclic organic carbonate;
a linear organic carbonate; and
the additive component of any preceding claim, and
an alkali salt.

8. The electrolyte solution of claim 7, wherein at least one of
the linear ester is selected from a group comprising methyl acetate, ethyl acetate, and methyl propionate,
the cyclic organic carbonate is selected from a group comprising ethylene carbonate and propylene carbonate, fluoroethylene carbonate, di-fluoroethylene carbonate, and vinylene carbonate; and
the linear organic carbonate is selected from a group comprising dimethyl carbonate, methyl ethyl carbonate, and diethyl carbonate.

9. The electrolyte solution of claim 7 or 8, wherein the alkali salt comprises lithium hexafluorophosphate (LiPF₆), lithium bis(fluorosulfonyl)imide (LiFSi), lithium bis-oxalatoborate (LiBOB), lithium difluorooxalatoborate (LiDFOB), lithium bis-trifluoromethansulfonate (LiTFSi), sodium hexafluorophosphate (NaPF₆), sodium bis(fluorosulfonyl)imide (NaFSi), sodium bis-trifluoromethansulfonate (NaTFSi), or a mixture thereof.

10. The electrolyte solution of any of claims 7 to 9, wherein at least one of:
a linear ester concentration is in a range of from about 0 wt% to about 80 wt% of the solvent;
a cyclic organic carbonate concentration ranges from about 0 wt% to about 40 wt% of the solvent; and
a linear organic carbonate concentration ranges from 2 wt% to about 95 wt% of the solvent.

11. An energy storage device comprising:
a cathode;
an anode;
a separator between the cathode and the anode; and
an electrolyte solution comprising:
a solvent comprising:
at least one of:
a linear ester;
a cyclic organic carbonate; and
a linear organic carbonate;
the additive component of claims 1 to 6; and
an alkali salt.

12. The energy storage device of claim 11, wherein the cathode is selected from a group comprising:
LiNi_{0.42}Mn_{0.42}Co_{0.16}O₂ (NMC442), LiNi_{0.65}Mn_{0.15}Co_{0.20}O₂ (Ni65),
LiNi_{0.91}Mn_{0.045}Co_{0.045}O₂ (Ni91), LiNi_{0.63}Mn_{0.31}Co_{0.06}O₂ (NM64), and
LiNiₓCo_{y}Al_{1-x-y}O₂ where x + y + z = 1 (NCA), and
LiNiₓMn_{y}Co_{1-x-y}O₂ (NMC) where x + y + z = 1, LiFePO₄ (LFP),
LiFeₓMn₁₋ₓPO₄ (LMFP), LiNiₓMn₂₋ₓO₄ (LNMO), LiMn₂O₄ (LMO),
and NaNiₓFe_{y}Mn_{z}O₂, where x + y + z = 1 (NFM).

13. The energy storage device of claim 11 or 12, wherein an anode is selected from a group comprising:
graphite; hard carbon; a Si material comprising Si, SiO, Li_{y}SiOₓ, SiC or any combination of graphite and Si material; Sn or any combination of hard carbon and Sn, Li₄Ti₅O₁₂ (LTO); Na₂Ti₆O₁₃ (NTO); and NaTi₂(PO₄)₃ (NTP), and/or
wherein the separator comprises a material selected from a group consisting of polyethylene (PE), polypropylene (PP), polyethylene-polypropylene multi-layer (PP/PE/PP), polyvinylidene fluoride (PVDF), anodized alumina (AAO), polyethylene oxide (PEO), polyvinyl alcohol (PVA), β"-alumina, Na₃Zr₂Si₂PO₁₂ (NASICON), and zeolite A.

14. The energy storage device of any of claims 11 to 13, having an operating temperature of between about -40 to about 90°C.

15. An electrolyte solution comprising:
a solvent comprising:
methyl acetate in a range of from about 0 wt% to about 50 wt% of the solvent;
ethylene carbonate in a range of from about 0 wt% to about 35 wt% of the solvent;
dimethyl carbonate in a range of from about 0 wt% to about 25 wt% of the solvent.
